# EUROPEAN PATENT SPECIFICATION

(11) **EP 4 444 709 B1**
(45) Date of publication and mention of the grant of the patent: **25.02.2026**
(21) Application number: 23748939.8
(22) Date of filing: 28.06.2023
(51) Int. Cl.: C07D 401/14, C07F 5/06, C07F 5/00, C07F 13/00, A61K 51/04

(54) **FAP-ALPHA SPECIFIC TUMOR DIAGNOSTIC IMAGING AGENT**
FAP-ALPHA-SPEZIFISCHES TUMORDIAGNOSTISCHES BILDGEBUNGSMITTEL
AGENT D'IMAGERIE DIAGNOSTIQUE DE TUMEUR SPÉCIFIQUE DE FAP-ALPHA

(30) Priority: 20.07.2022 CN 202210851703
(43) Date of publication of application: 16.10.2024
(73) Proprietor: Jiaxing Pharmadax Genesis Pharmaceutical Technology Co., Ltd., Jiaxing City, Zhejiang Province 314303 (CN)
(72) Inventor: WANG, Chun, Beijing 100027 (CN); NIAN, Jinxing, Beijing 100027 (CN)
(74) Representative: AWA Denmark A/S
(86) International application number: PCT/CN2023/103382
(87) International publication number: WO 2024/016976

(56) References cited:
- WO-A1-2019/154886
- CN-A- 111 991 570

## Description

This application claims the benefit of and priority to CN202210851703.X as filed on July 20, 2022.

### TECHNICAL FIELD

The present invention relates to a diagnostic preparation, in particular relates to a FAP-α specific tumor diagnostic imaging agent and its use in the diagnosis of a disease characterized by overexpression of fibroblast activation protein α (FAP-α) in a subject in need thereof.

### BACKGROUND

Tumor is the second leading cause of disease death. Early diagnosis, early treatment, and individualized comprehensive treatment of tumor are effective measures for reducing tumor mortality. With the continuous exploration of the oncogenesis mechanism, it has been found that cancer-associated fibroblasts (CAPs), one of the major stromal cells present in the tumor microenvironment (accounting for more than 90% in some tumor stroma) are involved in almost all stages of oncogenesis, and are closely related to tumor growth, migration and progression, which has become an attractive research target for tumor diagnosis and anti-tumor treatment.

A distinguishing feature of CAFs is the expression of fibroblast activation protein α (FAP-α) . FAP-α is a crucial protein of CAFs, a type II membrane bound glycoprotein belonging to the dipeptidyl peptidase 4 (DPP4) family with the molecular weight of 97kDa. FAP-α has both dipeptidyl peptidase and endopeptidase activity. The endopeptidase activity distinguishes FAP-α from the other members of the DPP4 family. FAP-α is selectively expressed on the surface of CAFs of more than 90% of epithelial carcinomas including breast cancer, ovarian cancer, lung cancer, colon cancer, rectal cancer, gastric cancer, pancreatic cancer, cutaneous melanoma, etc. FAP-α is not or only at insignificant levels expressed in fibroblasts of normal tissues, but is appeared transitorily in wound healing tissues or is expressed transitorily during embryogenesis, which makes FAP-α to be an attractive target for biology research of tumor stromal cells as well as tumor diagnosis and anti-tumor therapy.

In recent years, the research on FAP-α targeted imaging agent in tumor diagnosis has progressed rapidly. Uwe Haberkorn et al. developed a series of imaging agents and radiation targeted therapeutic agents for PET, MRI, SPECT imaging based on existing FAP-α small molecular inhibitors. WO2019154886A1 disclosed ligand compounds FAPI-02, FAPI-04, FAPI-19, FAPI-34, FAPI-42, FAPI-46, etc. for imaging agents, and meanwhile disclosed complex compounds for imaging formed by the above ligand compounds and a radionuclide including the complex compounds of ⁶⁸Ga-FAPI-02, ⁶⁸Ga-FAPI-04, ⁶⁸Ga-FAPI-46, Al¹⁸F-FAPI-42, etc. for PET imaging, and ^{99m}Tc-FAPI-34, ²⁰³Pb-FAPI-32, etc. for SPECT imaging. Jia Bing et al. disclosed complex compounds labeled by the radionuclide 99mTc 99mTc-HFAPi and 99mTc-HpFAPi in CN111991570A. By structural modification on the chelator and linker of the complex compound, ^{99m}Tc-HFAPi and ^{99m}Tc-HpFAPi exhibit more excellent in vivo biodistribution, higher tumor uptake, tumor/organ uptake ratios, and better tumor imaging capability than ^{99m}Tc-FAPI-34 that exhibits the best effect in the prior art. Chen Haojun et al. disclosed a ⁶⁸Ga labeled PET imaging agent for tumor diagnosis based on dimeric FAPI-46, which exhibits more excellent tumor uptake and retention than ⁶⁸Ga-FAPI-46 based on monomeric FAPI, however the PET imaging agent labeled by ⁶⁸Ga for tumor diagnosis takes lysine as the linkage skeleton, which leads to an asymmetrical molecular structure and redundant synthesis route.

With the deepening of the research on FAP-α targeted imaging agents, improvements have been made on the in vivo distribution, tumor uptake, imaging effect, and the like of such type of imaging agents, however, it still cannot meet the actual clinical requirement. There are still huge and urgent need in clinical for imaging agents having more excellent biodistribution, higher tumor uptake ratio, more improved imaging capability so far. It is also necessary to simplify the synthesis route of this type of imaging agents in order to enhance production efficiency and reduce production costs.

### SUMMARY

In order to address the above problem, the present invention provides a compound of Formula (IC), 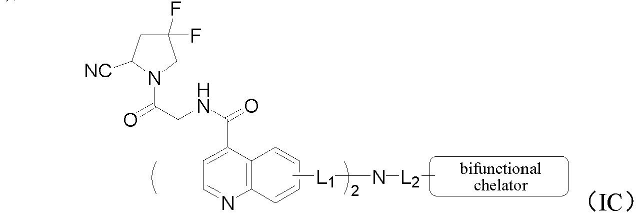 wherein,
L₁ is a functionalized linker of -X₁-CO-C₁₋₆alkylene (-X₂-C₁₋₆ alkylene)ₘ-;
L₂ is a functionalized linker of -CO-C₁₋₆alkylene-(X₃-C₁₋₆alkylene-)ₙ-X₄-;
X₁, X₂, X₃, X₄ are each independently selected from the group consisting of O, S, NH, and NCH₃; the C₁₋₆alkylene is optionally substituted by halo, OH, NH₂, oxo, and/or cyano;
m, n are each an integer selected from 0, 1, 2, 3, 4, 5, 6, 7, 8, 9, or 10;
the bifunctional chelator is selected from the group consisting of
6-(2-(sulfobenzylidene)hydrazinyl)nicotinic acid (HYNIC), mercaptoacetyldiglycine (MAG₂), mercaptoacetyltriglycine (MAG₃),
1,4,7,10-tetraazacyclododecane-1,4,7,10-tetraacetic acid (DOTA), 1,4,7-triazacyclononane-1,4,7-triacetic acid (NOTA), 1,4,8,11-tetraazacyclotetradecane-1,4,8,11-tetraacetic acid (TETA), ethylenediaminetetraacetic acid (EDTA), diethylenetriamine-N,N,N',N',N''-pentaacetic acid (DTPA), 3,6,9,15-tetraazabicyclo[9.3.1]pentadecane-1(15),11,13-triene-3,6,9-triacetic acid (PCTA), RESCA, 1,4,7-triazacyclononane-1-glutaric acid-4,7-diacetic acid (NOTA-GA), 1,4,7,10-tetrazazcyclododecane-1-glutaric acid-4,7,10-triacetic acid (DOTA-GA), 1,4,8,11-tetraazabicyclo[6,6,2]hexadecane-4,11-diacetic acid (CB-TE2A), 1,8-diamino-3,6,10,13,16,19-hexazabicyclo[6,6,6]eicosane (DiAmSar), 1-(4-isothiocyanatophenyl) -3-[6,17-dihydroxy-7,10,18,21-tetraoxo-27-(N-acetylhydroxylamino) -6,11,17,22-tetraazaheptacosane] (DFO) ;
or a pharmaceutically acceptable salt, stereoisomer thereof.

Specifically, the bifunctional chelator of the compound of formula (IC) is selected from the group consisting of 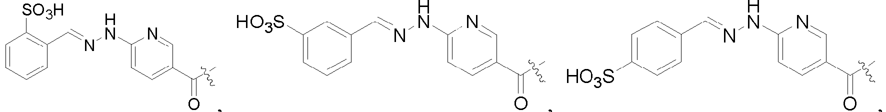 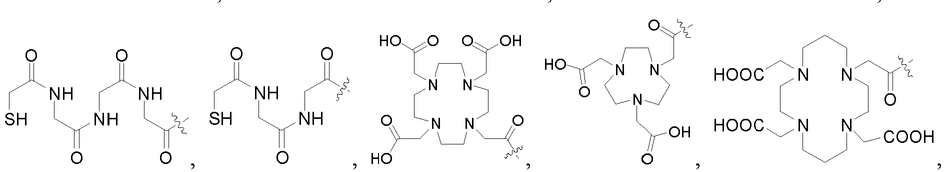 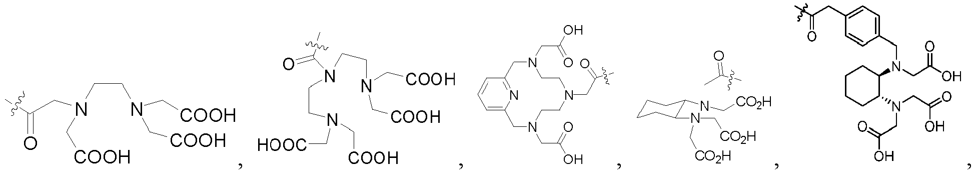 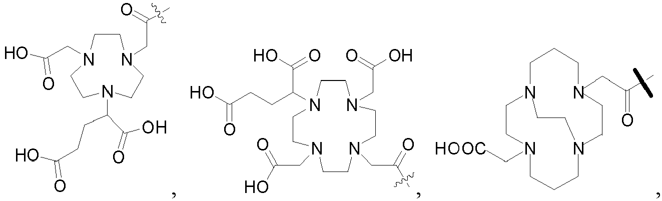 and

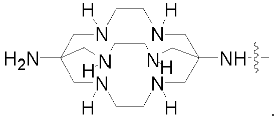

In one further embodiment, the compound of the present invention is the compound of formula (IC-I), (IC-II), (IC-III), or (IC-IV) 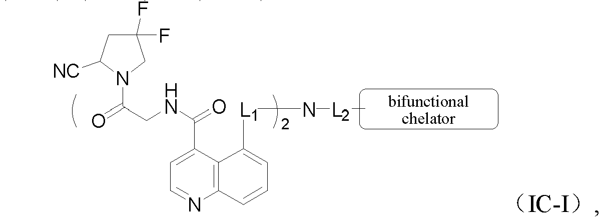 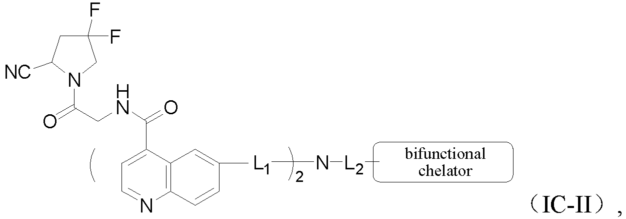 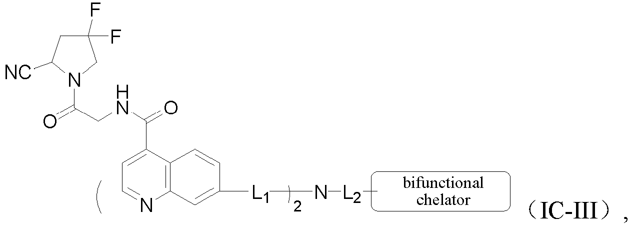 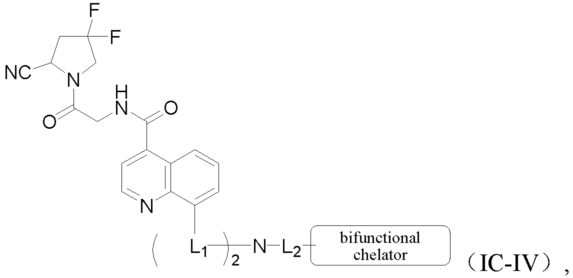
or a pharmaceutically acceptable salt, stereoisomer thereof;
wherein L₁ and L₂, and the bifunctional chelator are as defined herein.

In one further embodiment, the compound of the present invention is the compound of formula (ID) 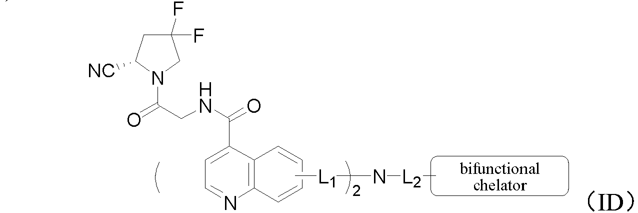
or a pharmaceutically acceptable salt, stereoisomer thereof;
wherein L₁ and L₂, and the bifunctional chelator are as defined herein.

In one further embodiment, the compound of the present invention is the compound of formula (ID-I), (ID-II), (ID-III), or (ID-IV) 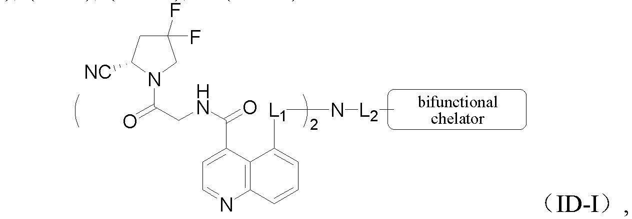 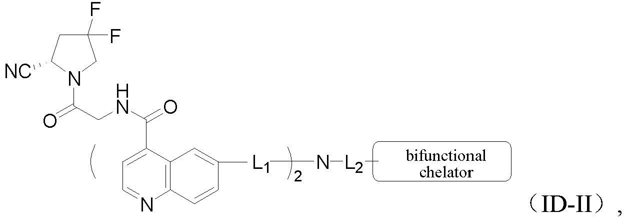 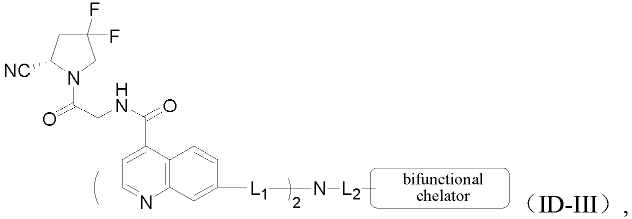 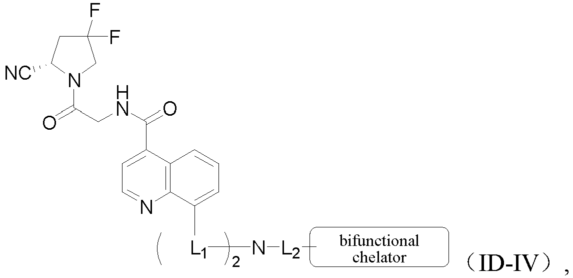
or a pharmaceutically acceptable salt, stereoisomer thereof;
wherein L₁ and L₂, and the bifunctional chelator are as defined herein.

In one specific embodiment, L₁ of formula(IC), (IC-I), (IC-II), (IC-III), (IC-IV), (ID), (ID-I), (ID-II), (ID-III), and (ID-IV) of the present invention is the functionalized linker selected from the group consisting of -NHCO-C₁₋₆alkylene(-O-C₁₋₆alkylene)ₘ-, -NHCO-C₁₋₆alkylene(-NH-C₁₋₆alkylene)ₘ-, -NHCO-C₁₋₆alkylene(-NCH₃-C₁₋₆alkylene)ₘ-, -NCH₃-CO-C₁₋₆ alkylene(-O-C₁₋₆alkylene)ₘ-, -NCH₃-CO-C₁₋₆alkylene(-NH-C₁₋₆alkylene)ₘ-, -NCH₃-CO-C₁₋₆alkylene(-NCH₃ -C₁₋₆alkylene)ₘ-, -OCO-C₁₋₆alkylene(-O-C₁₋₆alkylene)ₘ-, -OCO-C₁₋₆alkylene(-NH-C₁₋₆alkylene)ₘ-, -OCO-C₁₋₆alkylene(-NCH₃-C₁₋₆alkylene)ₘ-, wherein the alkylene is optionally substituted by halo, OH, NH₂, oxo, and/or cyano, m is an integer of 1, 2, 3, 4, 5, or 6.

In one preferable embodiment, L₁ is the functionalized linker selected from the group consisting of -NHCO-C₁₋₃alkylene(-O-C₁₋₃alkylene)ₘ-, -NHCO-C₁₋₃alkylene(-NH-C₁₋₃alkylene)ₘ-, -NHCO-C₁₋₃alkylene(-NCH₃-C₁₋₃alkylene)ₘ-, -NCH₃-CO-C₁₋₃alkylene(-O-C₁₋₃alkylene)ₘ-, -NCH₃-CO-C₁₋₃alkylene(-NH-C₁₋₃alkylene)ₘ-, -NCH₃-CO-C₁₋₃alkylene(-NCH₃-C₁₋₃alkylene)ₘ-, -OCO-C₁₋₃alkylene(-O-C₁₋₃alkylene)ₘ-, -OCO-C₁₋₃alkylene(-NH-C₁₋₃alkylene)ₘ-, -OCO-C₁₋₃alkylene(-NCH₃-C₁₋₃alkylene)ₘ-, wherein the alkylene is optionally substituted by halo, OH, NH₂, oxo, or cyano, m is an integer of 1, 2, 3, 4, 5, or 6.

In one more preferable embodiment, L₁ is the functionalized linker selected from the group consisting of -NHCO-CH₂CH₂(-O-CH₂CH₂)ₘ-, -NHCO-CH₂CH₂(-NH-CH₂CH₂)ₘ-, -NHCO-CH₂CH₂(-NCH₃-CH₂CH₂)ₘ-, -NCH₃-CO-CH₂CH₂(-O-CH₂CH₂)ₘ-, -NCH₃-CO-CH₂CH₂(-NH-CH₂CH₂)ₘ-, -NCH₃-CO-CH₂CH₂(-NCH₃-CH₂CH₂)ₘ-, -OCO-CH₂CH₂(-O-CH₂CH₂)ₘ-, -OCO-CH₂CH₂(-NH-CH₂CH₂)ₘ-, -OCO-CH₂CH₂(-NCH₃-CH₂CH₂)ₘ-, wherein said CH₂CH₂ is optionally substituted by halo, OH, NH₂, oxo, and/or cyano, m is an integer of 1, 2, 3, 4, 5, or 6.

In the most preferable embodiment, L₁ is the functionalized linker of -NHCO-CH₂CH₂-(-O-CH₂CH₂)₄-.

In a specific embodiment, L₂ of formula (IC), (IC-I), (IC-II), (IC-III), (IC-IV) , (ID), (ID-I), (ID-II), (ID-III), or (ID-IV) of the present invention is the functionalized linker selected from the group consisting of -CO-C₁₋₆alkylene-(NH-C₁₋₆alkylene-)ₙ-NH-, -CO-C₁₋₆alkylene-(NH-C₁₋₆alkylene-)ₙ-NCH₃-, -CO-C₁₋₆alkylene-(NH-C₁₋₆alkylene-)ₙ-O-, -CO-C₁₋₆alkylene-(O-C₁₋₆alkylenev-)ₙ-NH-, -CO-C₁₋₆alkylene-(O-C₁₋₆alkylene-)ₙ-NCH₃-, -CO-C₁₋₆alkylene-(O-C₁₋₆alkylene-)ₙ-O-, -CO-C₁₋₆alkylene-(NCH₃-C₁₋₆alkylene-)ₙ-NH-, -CO-C₁₋₆alkylene-(NCH₃-C₁₋₆alkylene-)ₙ-NCH₃-, -CO-C₁₋₆alkylene-(NCH₃-C₁₋₆alkylene-)ₙ-O-, wherein the alkylene is optionally substituted by halo, OH, NH₂, oxo, and/or cyano, n is an integer of 1, 2, 3, 4, 5, or 6.

In a preferable embodiment, L₂ is the functionalized linker selected from the group consisting of -CO-C₁₋₃alkylene-(NH-C₁₋₃alkylene-)ₙ-NH-, -CO-C₁₋₃alkylene-(NH-C₁₋₃alkylene-)ₙ-NCH₃-, -CO-C₁₋₃alkylene-(NH-C₁₋₃alkylene-)ₙ-O-, -CO-C₁₋₃alkylene-(O-C₁₋₃alkylene-)ₙ-NH-, -CO-C₁₋₃alkylene-(O-C₁₋₃alkylene-)ₙ-NCH₃-, -CO-C₁₋₃alkylene-(O-C₁₋₃alkylene-)ₙ-O-, -CO-C₁₋₃alkylene-(NCH₃-C₁₋₃alkylene-)ₙ-NH-, -CO-C₁₋₃alkylene-(NCH₃-C₁₋₃alkylene-)ₙ-NCH₃-, -CO-C₁₋₃alkylene-(NCH₃-C₁₋₃alkylene-)ₙ-O-, wherein the alkylene is optionally substituted by halo, OH, NH₂, oxo, and/or cyano, n is an integer of 1, 2, 3, 4, 5, or 6.

In one more preferable embodiment, L₂ is the functionalized linker selected from the group consisting of -CO-CH₂CH₂-(NH-CH₂CH₂-)ₙ-NH-, -CO-CH₂CH₂-(NH-CH₂CH₂-)ₙ-NCH₃-, -CO-CH₂CH₂-(NH-CH₂CH₂-)ₙ-O-, -CO-CH₂CH₂-(O-CH₂CH₂-)ₙ-NH-, -CO-CH₂CH₂-(O-CH₂CH₂-)ₙ-NCH₃-, -CO-CH₂CH₂-(O-CH₂CH₂-)ₙ-O-, -CO-CH₂CH₂-(NCH₃-CH₂CH₂-)ₙ-NH-, -CO-CH₂CH₂-(NCH₃-CH₂CH₂-)ₙ-NCH₃-, -CO-CH₂CH₂-(NCH₃-CH₂CH₂-)ₙ-O-, wherein said CH₂CH₂ is optionally substituted by halo, OH, NH₂, oxo, and/or cyano, n is an integer of 1, 2, 3, 4, 5, or 6.

In one most preferable embodiment, L₂ is the functionalized linker of -CO-CH₂CH₂-(O-CH₂CH₂-)₄-NH-.

In one specific embodiment, the compound of the present invention is selected from the group consisting of: 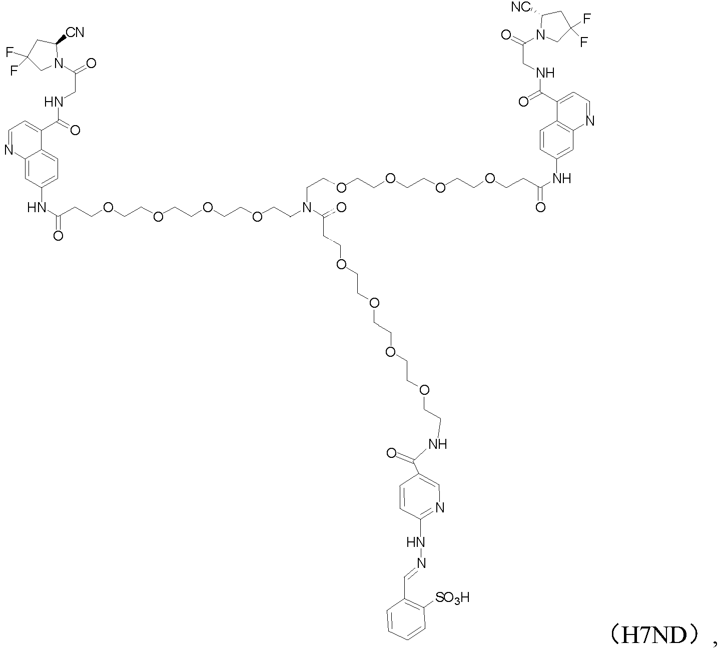 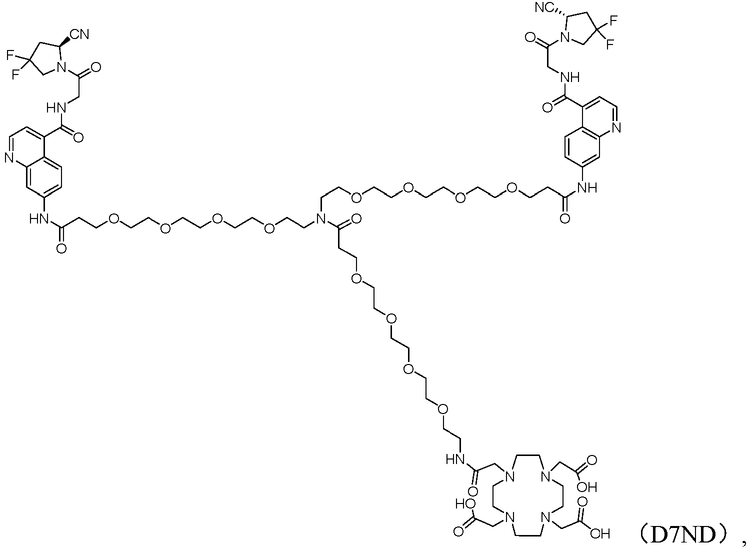 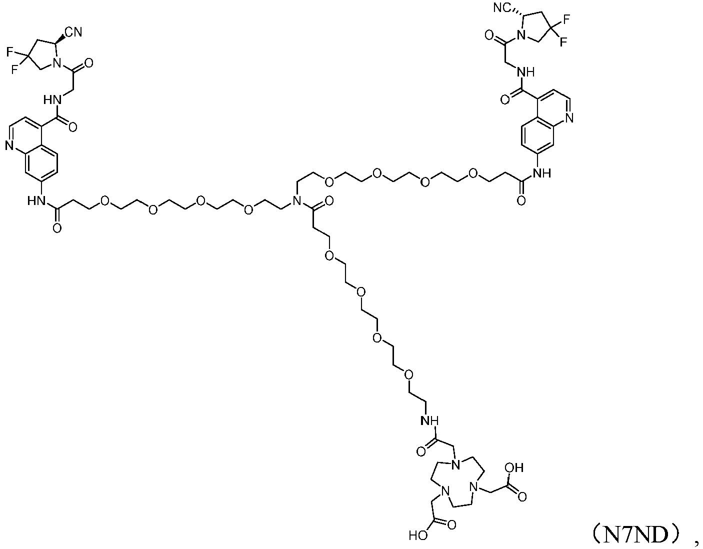 or a pharmaceutically acceptable salt, or stereoisomer thereof.

In another aspect, the present invention provides a kit, comprising the compound as described herein and a pharmaceutically acceptable carrier.

In one embodiment, the present invention provides a kit comprising the ligand compound of formula (IC), (IC-I), (IC-II), (IC-III), (IC-IV), (ID), (ID-I), (ID-II), , or (ID-IV), and a pharmaceutically acceptable carrier.

In one further embodiment, the present invention provides a kit comprising the ligand compound of H7ND, D7ND or N7ND as described above, and a pharmaceutically acceptable carrier, wherein the pharmaceutically acceptable carrier comprises, but not limited to, water, normal saline, buffered saline, etc. Preferably, the present invention provides a kit in the form of solution or freeze-dried powder, which optionally comprises one or more stabilizers, e.g. sodium chloride, silicate, phosphate buffer, acetate buffer, succinate buffer, ascorbic acid, gentisic acid, etc.

In one specific embodiment, the present invention provides a kit comprising the ligand compound H7ND, tris(hydroxymethyl)methylglycine(Tricine), Trisodium triphenylphosphine-3,3',3''-trisulfonate (TPPTS), and one or more stabilizers, as well as a pharmaceutically acceptable carrier. Preferably, the reagent within the kit is present in the form of freeze-dried powder, and is mixed with a radionuclide (e.g. ^{99m}Tc) prior to use.

In one specific embodiment, the present invention provides a kit comprising the ligand compound of D7ND, optionally one or more stabilizers, and a pharmaceutically acceptable carrier. The reagent within the kit is mixed with a radionuclide (e.g. ⁶⁸Ga) prior to use.

In one specific embodiment, the present invention provides a kit comprising the ligand compound of D7ND, optionally one or more stabilizers, and a pharmaceutically acceptable carrier. The reagent within the kit is mixed with a radionuclide (e.g. ¹⁷⁷Lu) prior to use.

In one specific embodiment, the present invention provides a kit comprising the ligand compound of D7ND, optionally one or more stabilizers, and a pharmaceutically acceptable carrier. The reagent within the kit is mixed with a radionuclide (e.g. ⁹⁰Y) prior to use.

In one specific embodiment, the present invention provides a kit comprising the ligand compound of N7ND, optionally one or more stabilizers, and a pharmaceutically acceptable carrier. The reagent within the kit is mixed with a radionuclide (e.g. ¹⁸F) prior to use.

In another aspect, the present invention provides a method for the preparation of a kit, comprising the step of mixing the ligand compound as described herein with a pharmaceutically acceptable carrier.

In one embodiment, the present invention provides a method for the preparation of a kit, comprising the step of mixing the ligand compound as described herein with one or more stabilizers, as well as a pharmaceutically acceptable carrier.

In one specific embodiment, the present invention provides a method for the preparation of a kit comprising the step of mixing the ligand compound H7ND as described herein with Tricine, TPPTS, and one or more stabilizers, as well as a pharmaceutically acceptable carrier to form a solution. Preferably, said method further comprises the step of freeze drying the solution.

In one specific embodiment, the present invention provides a method for the preparation of a kit, comprising the step of mixing the ligand compound D7ND, optionally one or more stabilizers, and a pharmaceutically acceptable carrier to form a solution.

In one specific embodiment, the present invention provides a method for the preparation of a kit, comprising the step of mixing the ligand compound N7ND, optionally one or more stabilizers, and a pharmaceutically acceptable carrier to form a solution.

In another aspect, the present invention provides a complex compound, comprising the compound of formula (IC) 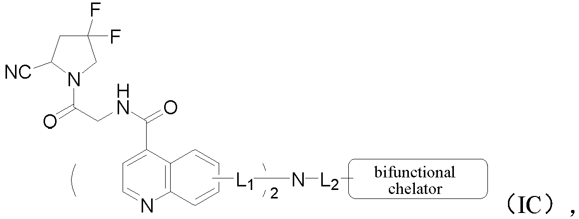 or a pharmaceutically acceptable salt, or stereoisomer thereof; and a radionuclide, wherein the radionuclide is conjugated to the bifunctional chelator of formula (IC) by a coordination bond; wherein L₁, L₂, and the bifunctional chelator are as defined herein.

In one embodiment, the present invention provides a complex compound, comprising the compound of formula (IC-I) 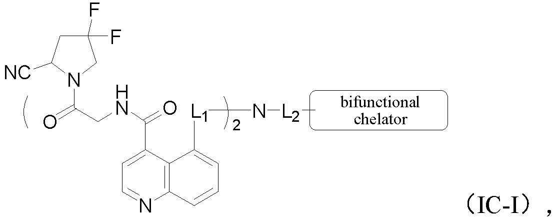 or a pharmaceutically acceptable salt, or stereoisomer thereof; and a radionuclide, wherein the radionuclide is conjugated to the bifunctional chelator of formula (IC-I) by a coordination bond; wherein L₁, L₂, and the bifunctional chelator are as defined herein.

In one embodiment, the present invention provides a complex compound, comprising the compound of formula (IC-II) 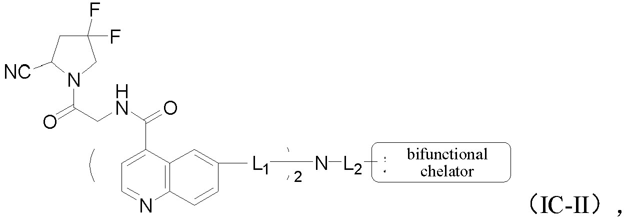 or a pharmaceutically acceptable salt, or stereoisomer thereof; and a radionuclide, wherein the radionuclide is conjugated to the bifunctional chelator of formula (IC-II) by a coordination bond; wherein L₁, L₂, and the bifunctional chelator are as defined herein.

In one embodiment, the present invention provides a complex compound, comprising the compound of formula (IC-III) 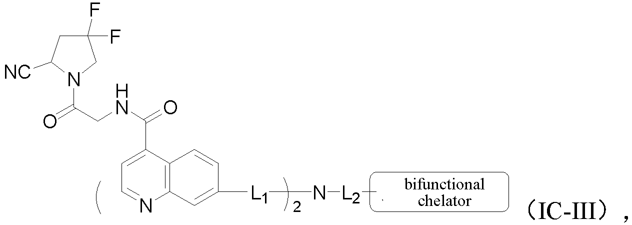 or a pharmaceutically acceptable salt, or stereoisomer thereof; and a radionuclide, wherein the radionuclide is conjugated to the bifunctional chelator of formula (IC-III) by a coordination bond; wherein L₁, L₂, and the bifunctional chelator are as defined herein.

In one embodiment, the present invention provides a complex compound, comprising the compound of formula (IC-IV) 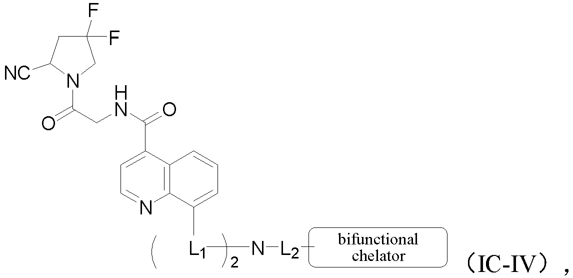 or a pharmaceutically acceptable salt, or stereoisomer thereof; and a radionuclide, wherein the radionuclide is conjugated to the bifunctional chelator of formula (IC-IV) by a coordination bond; wherein L₁, L₂, and the bifunctional chelator are as defined herein.

In one embodiment, the present invention provides a complex compound, comprising the compound of formula (ID) 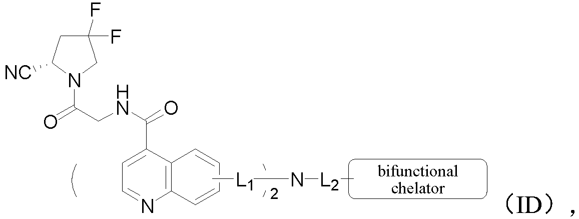 or a pharmaceutically acceptable salt, or stereoisomer thereof; and a radionuclide, wherein the radionuclide is conjugated to the bifunctional chelator of formula (ID) by a coordination bond; wherein L₁, L₂, and the bifunctional chelator are as defined herein.

In one embodiment, the present invention provides a complex compound, comprising the compound of formula (ID-I) 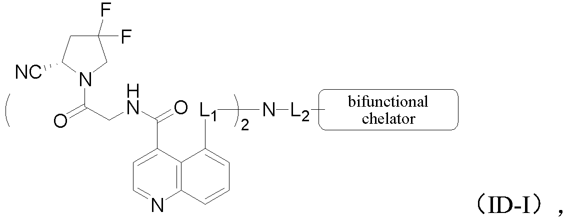 or a pharmaceutically acceptable salt, or stereoisomer thereof; and a radionuclide, wherein the radionuclide is conjugated to the bifunctional chelator of formula (ID-I) by a coordination bond; wherein L₁, L₂, and the bifunctional chelator are as defined herein.

In one embodiment, the present invention provides a complex compound, comprising the compound of formula (ID-II) 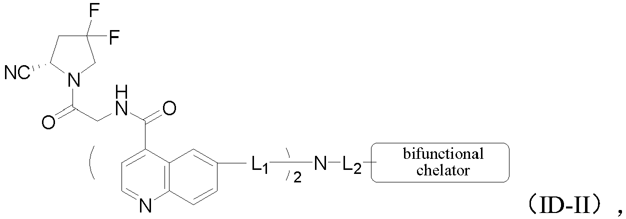 or a pharmaceutically acceptable salt, or stereoisomer thereof; and a radionuclide, wherein the radionuclide is conjugated to the bifunctional chelator of formula (ID-II) by a coordination bond; wherein L₁, L₂, and the bifunctional chelator are as defined herein.

In one embodiment, the present invention provides a complex compound, comprising the compound of formula (ID-III) 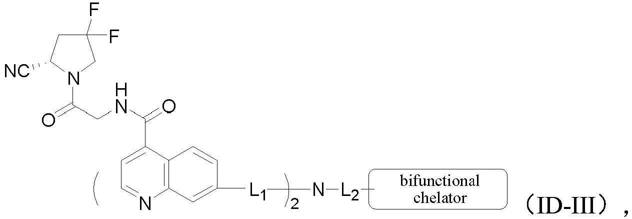 or a pharmaceutically acceptable salt, or stereoisomer thereof; and a radionuclide, wherein the radionuclide is conjugated to the bifunctional chelator of formula (ID-III) by a coordination bond; wherein L₁, L₂, and the bifunctional chelator are as defined herein.

In one embodiment, the present invention provides a complex compound, comprising the compound of formula (ID-IV) 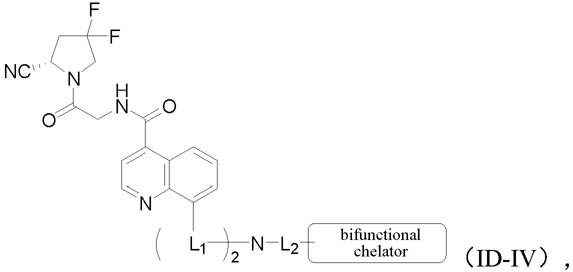 or a pharmaceutically acceptable salt, or stereoisomer thereof; and a radionuclide, wherein the radionuclide is conjugated to the bifunctional chelator of formula (ID-IV) by a coordination bond; wherein L₁, L₂, and the bifunctional chelator are as defined herein.

In one embodiment, the present invention provides a complex compound, comprising the compound of 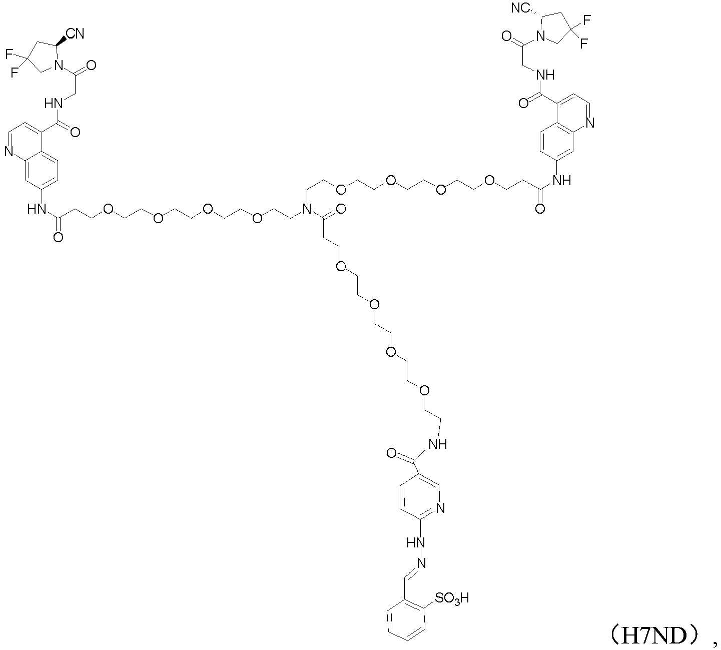 or 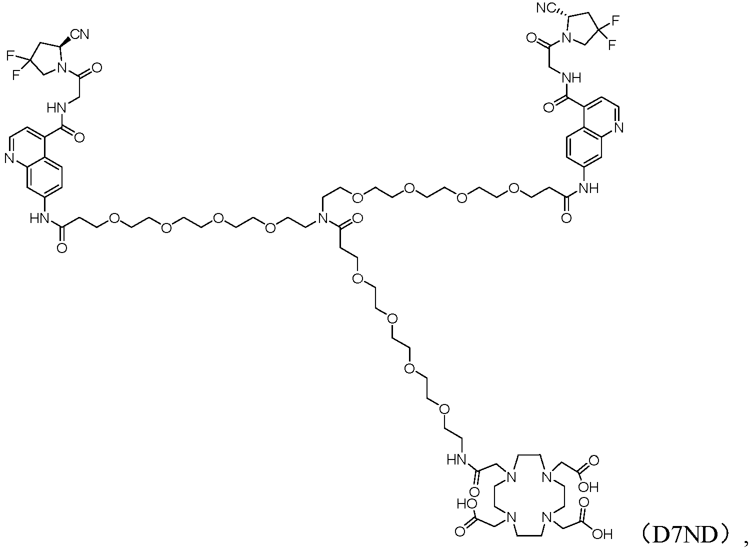 or 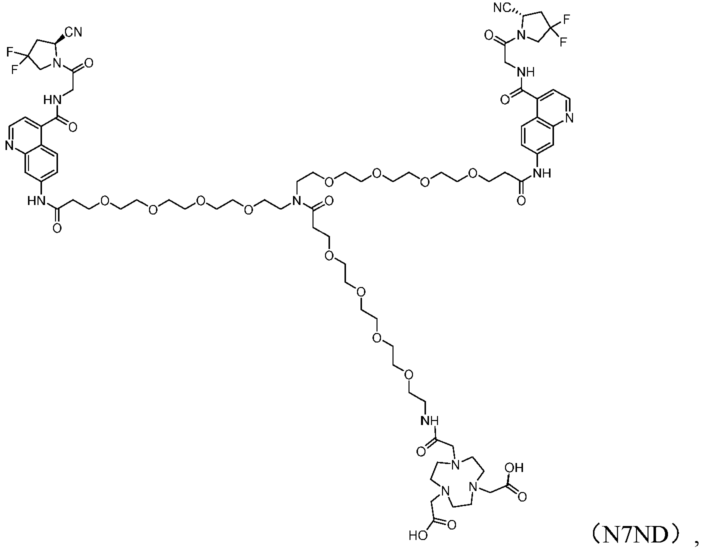 and a radionuclide, wherein the radionuclide is conjugated to the bifunctional chelator of compound H7ND or D7ND or N7ND by a coordination bond.

In one specific embodiment, the complex compound as described herein comprises a radionuclide comprising alpha radiation emitting isotopes, beta radiation emitting isotopes, gamma radiation emitting isotopes, Auger electron emitting isotopes, or X-ray emitting isotopes, etc.

In one preferable embodiment, the complex compound as described herein comprises a radionuclide comprising, but not limited to, ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁸Y, ⁹⁰Y, ¹⁴⁹Pm, ¹⁷⁷Lu, ⁴⁷Sc, ²¹²Bi, ²¹³Bi, ⁷²As, ¹²³I, ¹²⁴I, ¹³¹I, ²¹¹At, ²⁰¹Tl , ²¹²Pb, ⁶⁴Cu, ⁶⁷Cu, ¹⁹⁸Au, ²²⁵Ac, ²²³Ra, or ⁸⁹Sr, etc.

In one more preferable embodiment, the complex compound as described herein comprises a radionuclide comprising, but not limited to, ⁶⁸Ga, ^{99m}Tc, ¹⁷⁷Lu, ⁹⁰Y, or ¹⁸F, etc.

In one specific embodiment, the present invention provides a complex compound, comprising 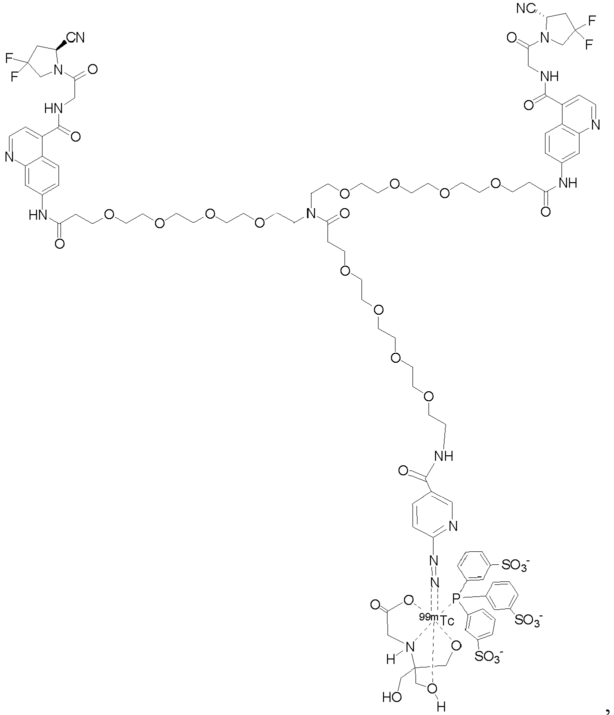 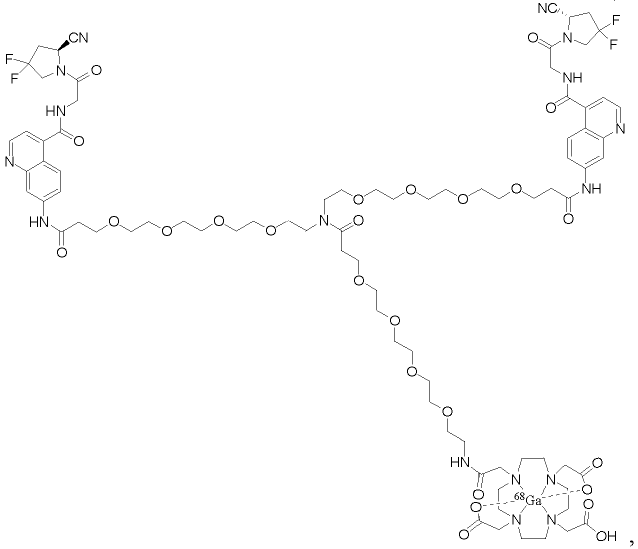 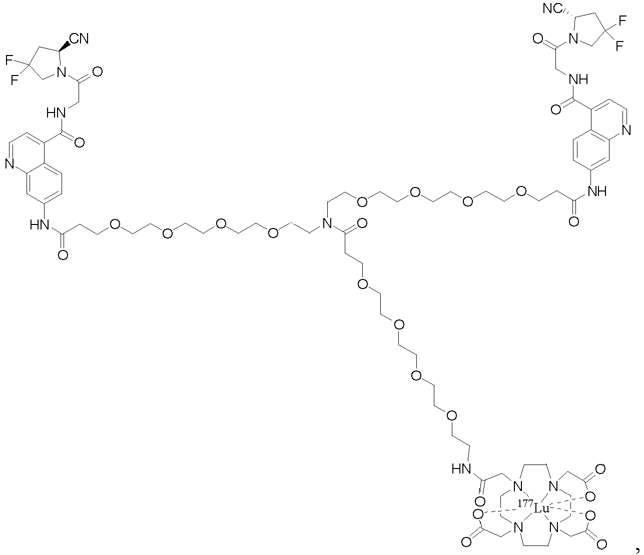 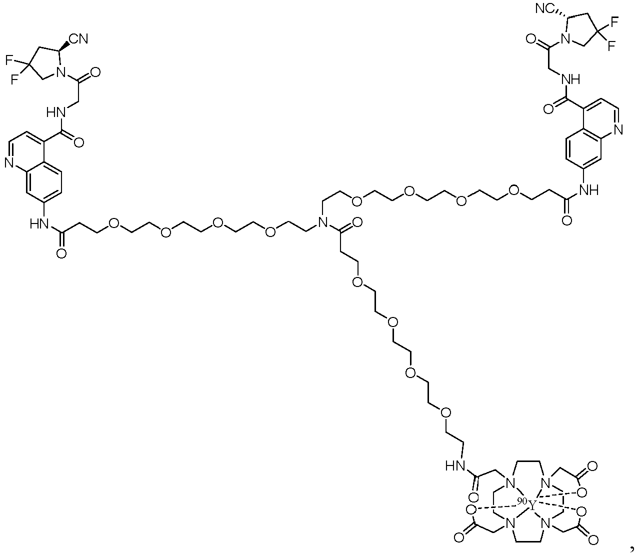 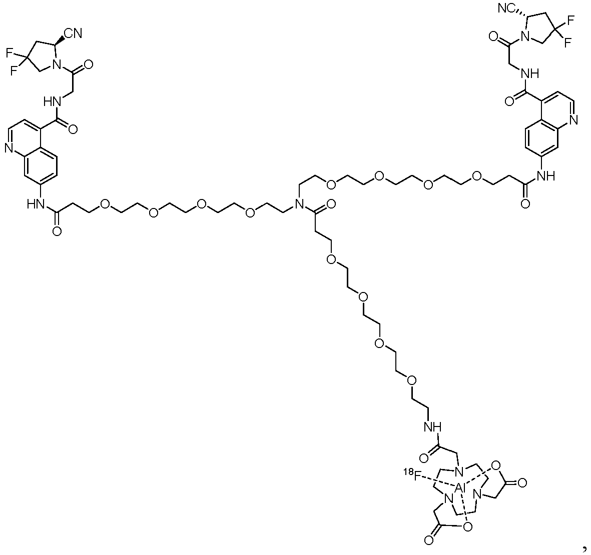 or a pharmaceutically acceptable salt, or stereoisomer thereof.

In another aspect, the present invention provides a diagnostic kit, comprising the complex compound as described herein and a pharmaceutically acceptable carrier.

In one embodiment, the present invention provides a diagnostic kit comprising the complex compound as described herein and a pharmaceutically acceptable carrier, wherein the pharmaceutically acceptable carrier comprises, but not limited to, water, normal saline, buffered saline, etc. Preferably, the present invention provides a diagnostic kit, which optionally comprises one or more stabilizers, e.g. sodium chloride, silicate, phosphate buffer, acetate buffer, succinate buffer, ascorbic acid, gentisic acid, etc.

In one specific embodiment, the present invention provides a diagnostic kit comprising the complex compound of ^{99m}Tc-H7ND, optionally one or more stabilizers such as sodium chloride, silicate, phosphate buffer, acetate buffer, succinate buffer, ascorbic acid, gentisic acid, etc, and a pharmaceutically acceptable carrier.

In one specific embodiment, the present invention provides a diagnostic kit comprising the complex compound of ⁶⁸Ga-D7ND, optionally one or more stabilizers such as sodium chloride, silicate, phosphate buffer, acetate buffer, succinate buffer, ascorbic acid, gentisic acid, etc, and a pharmaceutically acceptable carrier.

In one specific embodiment, the present invention provides a diagnostic kit comprising the complex compound of ¹⁷⁷Lu-D7ND, optionally one or more stabilizers such as sodium chloride, silicate, phosphate buffer, acetate buffer, succinate buffer, ascorbic acid, gentisic acid, etc, and a pharmaceutically acceptable carrier.

In one specific embodiment, the present invention provides a diagnostic kit comprising the complex compound of ⁹⁰Y-D7ND, optionally one or more stabilizers such as sodium chloride, silicate, phosphate buffer, acetate buffer, succinate buffer, ascorbic acid, gentisic acid, etc, and a pharmaceutically acceptable carrier.

In one specific embodiment, the present invention provides a diagnostic kit comprising the complex compound of Al¹⁸F-N7ND, optionally one or more stabilizers such as sodium chloride, silicate, phosphate buffer, acetate buffer, succinate buffer, ascorbic acid, gentisic acid, etc, and a pharmaceutically acceptable carrier.

In another aspect, the present invention provides a method for the diagnosis of a disease characterized by overexpression of fibroblast activation protein α (FAP-α) in a subject in need thereof, comprising the use of the complex compound as described herein or the kit comprising the same, and imaging.

In one preferable embodiment, the complex compound as described herein may be administered directly into the blood stream, into muscle, or into an internal organ. Suitable routes for such parenteral administration include intravenous, intraarterial, intraperitoneal, intrathecal, epidural, intracerebroventricular, intraurethral, intrasternal, intracranial, intratumoral, intramuscular and subcutaneous delivery. Depending upon the cancer type as described herein, and/or the route of administration, a wide range of permissible dosages are contemplated herein, including doses falling in the range from about 1 µg/kg to about 1 g/kg; preferably, from about 1 µg/kg to about 0.5 g/kg; more preferably, from about 1 µg/kg to about 0.1 g/kg; more preferably, from about 1 µg/kg to about 0.05 g/kg; more preferably, from about 1 µg/kg to about 0.01 g/kg; more preferably, from about 1 µg/kg to about 5 mg/kg; more preferably, from about 1 µg/kg to about 1 mg/kg; more preferably, from about 1 µg/kg to about 0.5 mg/kg; more preferably, from about 1 µg/kg to about 0.1 mg/kg; more preferably, from about 1 µg/kg to about 0.05 mg/kg, more preferably, from about 1 µg/kg to about 0.01 mg/kg.

In one preferable embodiment, imaging may be performed by any method suitable for determining one specific radionuclide, such as positron emission tomography (PET), single photon emission computed tomography (SPECT), etc.

In one preferable embodiment, the disease characterized by overexpression of fibroblast activation protein α (FAP-α) comprises, but not limited to, cancer, chronic inflammation, atherosclerosis, fibrosis, tissue remodeling, and keloid disorder.

In one preferable embodiment, the disease characterized by overexpression of fibroblast activation protein α (FAP-α) is cancer, which comprises, but not limited to, breast cancer, pancreatic cancer, small intestine cancer, colon cancer, rectal cancer, lung cancer, head and neck cancer, ovarian cancer, liver cancer, esophageal cancer, gastric cancer, hypopharynx cancer, nasopharynx cancer, larynx cancer, myeloma, bladder cancer, cholangiocarcinoma, renal carcinoma, neuroendocrine tumor, oncogenic osteomalacia, sarcoma, carcinoma of unknown primary, thymus carcinoma, glioma, astrocytoma, cervix carcinoma, prostate cancer, and testicular cancer.

In one more preferable embodiment, the disease characterized by overexpression of fibroblast activation protein α (FAP-α) is chronic inflammation, which comprises, but not limited to, rheumatoid arthritis, osteoarthritis, Crohn's disease, etc.

In one more preferable embodiment, the disease characterized by overexpression of fibroblast activation protein α (FAP-α) is fibrosis, which comprises, but not limited to, idiopathic pulmonary fibrosis, hepatic cirrhosis, etc.

In one more preferable embodiment, the disease characterized by overexpression of fibroblast activation protein α (FAP-α) is keloid disorder, which comprises, but not limited to, scar formation, keloid tumors, keloid scar, etc.

In another aspect, the present invention provides use of the complex compound as described herein in the manufacture of a diagnostic kit for the diagnosis of a disease characterized by overexpression of fibroblast activation protein α (FAP-α) in a subject in need thereof.

In one embodiment, the present invention provides use of the complex compound as described herein in the manufacture of a diagnostic kit for the diagnosis of a disease characterized by overexpression of fibroblast activation protein α (FAP-α) in a subject in need thereof, wherein the reagent comprised in the diagnostic kit is administered to the subject, and then imaging was performed on the subject.

In one preferable embodiment, imaging may be performed by any method suitable for determining one specific radionuclide, such as positron emission tomography (PET), single photon emission computed tomography (SPECT), etc.

In one preferable embodiment, the disease characterized by overexpression of fibroblast activation protein α (FAP-α) comprises, but not limited to, cancer, chronic inflammation, atherosclerosis, fibrosis, tissue remodeling, and keloid disorder.

In one more preferable embodiment, the disease characterized by overexpression of fibroblast activation protein α (FAP-α) is cancer, which comprises, but not limited to, breast cancer, pancreatic cancer, small intestine cancer, colon cancer, rectal cancer, lung cancer, head and neck cancer, ovarian cancer, liver cancer, esophageal cancer, gastric cancer, hypopharynx cancer, nasopharynx cancer, larynx cancer, myeloma, bladder cancer, cholangiocarcinoma, renal carcinoma, neuroendocrine tumor, oncogenic osteomalacia, sarcoma, carcinoma of unknown primary, thymus carcinoma, glioma, astrocytoma, cervix carcinoma, prostate cancer, and testicular cancer.

In one more preferable embodiment, the disease characterized by overexpression of fibroblast activation protein α (FAP-α) is chronic inflammation, which comprises, but not limited to, rheumatoid arthritis, osteoarthritis, Crohn's disease, etc.

In one more preferable embodiment, the disease characterized by overexpression of fibroblast activation protein α (FAP-α) is fibrosis, which comprises, but not limited to, idiopathic pulmonary fibrosis, hepatic cirrhosis, etc.

In one more preferable embodiment, the disease characterized by overexpression of fibroblast activation protein α (FAP-α) is keloid disorder, which comprises, but not limited to, scar formation, keloid tumor, keloid scar, etc.

In another aspect, the present invention provides the complex compound as described herein for use in the diagnosis of a disease.

In one embodiment, the present invention provides the complex compound as described herein for use in the diagnosis of a disease characterized by overexpression of fibroblast activation protein α (FAP-α) in a subject in need thereof, wherein the reagent comprised in the diagnostic kit is administered to the subject, and then imaging was performed on the subject.

In one specific embodiment, the present invention provides the complex compound as described herein for use in the diagnosis of a disease characterized by overexpression of fibroblast activation protein α (FAP-α) in a subject in need thereof, wherein the complex compound is administered to the subject, and then imaging was performed on the subject.

In one preferable embodiment, imaging may be performed by any method suitable for determining one specific radionuclide, such as positron emission tomography (PET), single photon emission computed tomography (SPECT), etc.

In one preferable embodiment, the disease characterized by overexpression of fibroblast activation protein α (FAP-α) comprises, but not limited to, cancer, chronic inflammation, atherosclerosis, fibrosis, tissue remodeling, and keloid disorder.

In one preferable embodiment, the disease characterized by overexpression of fibroblast activation protein α (FAP-α) is cancer, which comprises, but not limited to, breast cancer, pancreatic cancer, small intestine cancer, colon cancer, rectal cancer, lung cancer, head and neck cancer, ovarian cancer, liver cancer, esophageal cancer, gastric cancer, hypopharynx cancer, nasopharynx cancer, larynx cancer, myeloma, bladder cancer, cholangiocarcinoma, renal carcinoma, neuroendocrine tumor, oncogenic osteomalacia, sarcoma, carcinoma of unknown primary, thymus carcinoma, glioma, astrocytoma, cervix carcinoma, prostate cancer, and testicular cancer.

In one more preferable embodiment, the disease characterized by overexpression of fibroblast activation protein α (FAP-α) is chronic inflammation, which comprises, but not limited to, rheumatoid arthritis, osteoarthritis, Crohn's disease, etc.

In one more preferable embodiment, the disease characterized by overexpression of fibroblast activation protein α (FAP-α) is fibrosis, which comprises, but not limited to, idiopathic pulmonary fibrosis, hepatic cirrhosis, etc.

In one more preferable embodiment, the disease characterized by overexpression of fibroblast activation protein α (FAP-α) is keloid disorder, which comprises, but not limited to, scar formation, keloid tumors, keloid scar, etc.

### Beneficial Effect

The FAP-α specific tumor diagnostic imaging agent of the present invention is of the dimeric form. Compared with the ^{99m}Tc-HFAPi or ⁶⁸Ga-FAPi-04 imaging agent based on the monomeric FAP-α in the prior art, the imaging agent of the present invention has significantly higher FAP-α binding affinity and in vivo stability, higher tumor/normal tissue uptake ratio, and better imaging contrast, which is beneficial to the diagnosis of tiny tumor lesions, in particular when SPECT imaging is sensitive to the background signal noise.

Compared with ^{99m}Tc-HFAPi or ⁶⁸Ga-FAPi-04 imaging agent based on the monomeric FAP-α in the prior art, the FAP-α specific tumor diagnostic imaging agent of the present invention has significantly less in vivo retention time, which may reduce radiation exposure to a patient, and lower the risk of adverse reaction to a patient caused by radiation.

Compared with ⁶⁸Ga labeled tumor diagnostic PET imaging agent based on FAP-46 of dimeric form as described above, the FAP-α specific tumor diagnostic imaging agent of the present invention takes nitrogen atom as the linkage skeleton and has a symmetric molecular structure as a whole, which makes the synthesis concise and efficient, significantly improves the production efficiency, and lower the production cost. Furthermore, compared with ⁶⁸Ga labeled tumor diagnostic PET imaging agent based on FAP-46 of dimeric form as described above, the FAP-α specific tumor diagnostic imaging agent of the present invention achieves significantly better technical effect in the aspect of improving FAP-α binding affinity and tumor uptake.

### BRIEF DESCRIPTION OF THE DRAWINGS

The drawings forming a part of the disclosure are adopted to provide further understanding of the disclosure. The drawings together with the examples of the present invention are adopted to explain the present invention and not intended to form improper limits to the present invention. In the drawings:

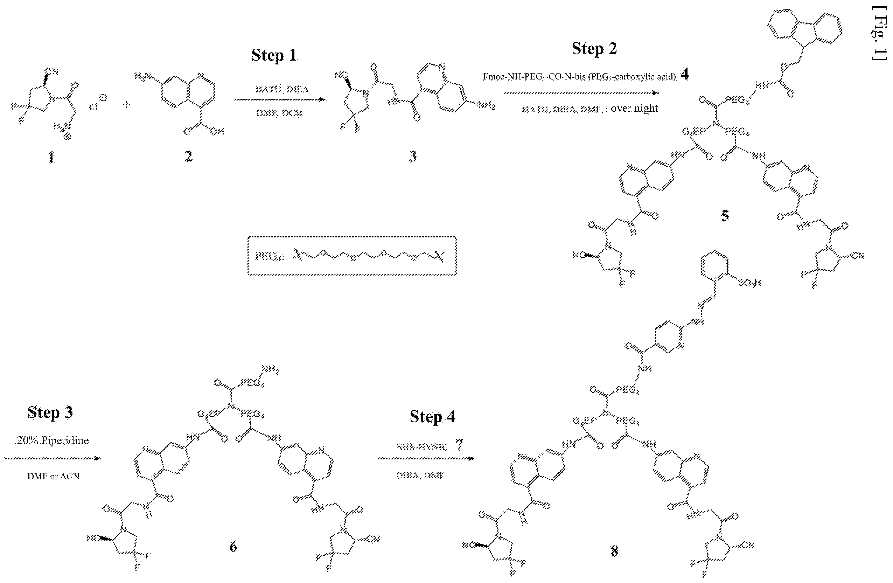

Figure 1 shows the synthesis route of the complex compound H7ND.
Figure 2 shows the mass spectrography analysis spectrum of the complex compound H7ND.
Figure 3 shows the mass spectrography analysis spectrum of the complex compound D7ND.
Figure 4 shows the quality control analysis of the complex compound ⁶⁸Ga-D7ND, wherein Figure 4A shows the measurement results of labeling yield of ⁶⁸Ga-D7ND, Figure 4B shows the measurement results of radiochemical purity of ⁶⁸Ga-D7ND after purification.
Figure 5 shows in vitro cell binding assay of the complex compound ^{99m}Tc-H7ND.
Figure 6 shows the experiment results of in vivo statibility and metabolism of the complex compound ^{99m}Tc-H7ND in normal mice, wherein Figure 6A shows the results of in vivo metabolic stability test of the complex compounds ^{99m}Tc-HFAPi and ^{99m}Tc-H7ND in mice, Figure 6B shows the results of in vivo retention test of the complex compound ^{99m}Tc-HFAPi and ^{99m}Tc-H7ND in mice.
Figure 7 shows SPECT/CT imaging of the complex compound ^{99m}Tc-H7ND in U87MG tumor-bearing mouse model.
Figure 8 shows the results of biodistribution comparison of the complex compounds ^{99m}Tc-H7ND and ^{99m}Tc-HFAPi in U87MG tumor-bearing mouse model, wherein Figure 8A shows the results of biodistribution of ^{99m}Tc-H7ND in U87MG tumor-bearing mouse model, Figure 8B shows the tumor/normal organ uptake ratio of ^{99m}Tc-H7ND in U87MG tumor-bearing mouse model; Figure 8C shows the results of biodistribution of ^{99m}Tc-HFAPi in U87MG tumor-bearing mouse model, Figure 8D shows the tumor/normal organ uptake ratio of ^{99m}Tc-HFAPi in U87MG tumor-bearing mouse model.
Figure 9 shows comparison of Micro-PET imaging of complex compounds ⁶⁸Ga-D7ND and ⁶⁸Ga-FAPI-04 in U87MG tumor-bearing mouse model.
Figure 10 shows ¹⁸F-FDG-PET and ^{99m}Tc-H7ND-SPECT imaging of patients with lung tumor, wherein Figure 10A shows ¹⁸F-FDG-PET/CT imaging, Figure 10B shows ^{99m}Tc-H7ND-SPECT imaging.
Figure 11 shows the ^{99m}Tc-H7ND-SPECT imaging of patients with lung tumor.

### DETAILED DESCRIPTION

### DEFINITION

As used in the present specification, the following words and phrases are generally intended to have the meanings as set forth below, except to the extent that the context in which they are used indicates otherwise.

As used herein, the articles "a" and "an" refer to one or more than one (i.e., at least one) of the grammatical object of the article. By way of example, "an element" means one element or more than one element.

As used herein, the term "halo" refers to fluoro or fluorine, chloro or chlorine, bromo or bromine, and iodo or iodine.

As used herein, the term "pharmaceutically acceptable salt" refers to a salt that remains bioavailability and property of a given compound, and the salt is not biologically or otherwise undesirable. A pharmaceutically acceptable salt may be prepared from inorganic acids and organic acids. Salts derived from organic acids comprise hydrochloride, hydrobromide, sulfate, nitrate, phosphate, carbonate, bisulfate, biphosphate, dihydrophosphate, bicarbonate, etc. Salts derived from organic acids comprise formate, acetate, propionate, glycollate, pyruvate, oxalate, malate, malonate, succinate, maleate, fumarate, tartrate, citrate, benzoate, cinnamate, mandelate, mesylate, ethyl sulfonate, tosylate, salicylate, etc.

As used herein, the term "amino protecting group" is well understood by the person skilled in synthetic organic chemistry as a moiety that can be selectively installed onto and removed from a suitable amine functional group. The field of protecting group methodology is advanced, and many amine protecting groups, and methods for using them, are well known in the art, such as those described in the authoritative treatise on the subject, P. G. M. Wuts and T. W. Greene, Greene's Protective Groups in Organic Synthesis, 4th Edition (Wiley, 2006).

As used herein, the terms "optional" or "optionally" mean that the subsequently described event or circumstance may or may not occur, and that the description includes instances where said event or circumstance occurs and instances in which it does not occur.

As used herein, the term "stereoisomer" refers to compounds which have identical chemical constitution and connectivity, but different orientations of their atoms in space that cannot be interconverted by rotation about single bonds. "Stereoisomer" comprises "diastereomer" and "enantiomers". "Diastereomer" refers to a stereoisomer with two or more centers of chirality and whose molecules are not mirror images of one another. Diastereomers have different physical properties, e.g. melting points, boiling points, spectral properties, and reactivities. Mixtures of diastereomers may be separated under high resolution analytical procedures such as crystallization, electrophoresis and chromatography. "Enantiomers" refer to two stereoisomers of a compound which are non-superimposable mirror images of one another.

### General Synthesis

The compounds of the present invention may be prepared using methods disclosed herein and routine modifications thereof which will be apparent given the disclosure herein and methods well known in the art. Typical embodiments of compounds in accordance with the present invention may be synthesized using the general reaction schemes described below. It will be apparent given the description herein that the general schemes may be altered by substitution of the starting materials with other materials having similar structures to result in products that are correspondingly different. Starting materials are typically obtained from commercial sources or synthesized using published methods.

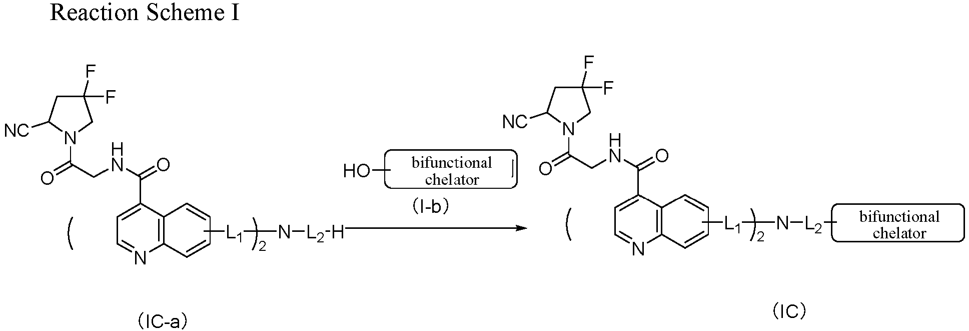

The compound of formula (IC-a) is condensed with the compound of formula (I-b) under conditions suitable for the formation of an amide. For example, to a mixture of the compounds of formula (IC-a) and formula (I-b) in an inert solvent (e.g. dimethyl sulfoxide, N,N-dimethyl formamide, acetonitrile, tetrahydrofuran, methylene chloride) are added a condensing agent (e.g. 2- (7-aza-1H-benzotriazol-1-yl) -1,1,3,3-tetramethyluronium hexafluorophosphate (HATU), O-benzotriazol-N,N,N',N'-tetramethyluronium hexafluorophosphate (HBTU), dicyclohexylcarbodiimide(DCC), N,N'-diisopropylcarbodiimide(DIC), 1-(3-dimethylaminopropyl)-3-ethylcarbodiimide(EDCI), ethyl cyanoglyoxylate-2-oxime), and a base (e.g. N-methylmorpholine, 4-dimethylaminopyridine, triethylamine, diisopropylethylamine, etc) for reaction to give the compound of formula (IC), wherein, variables of L₁, L₂, and the bifunctional chelator are as defined herein.

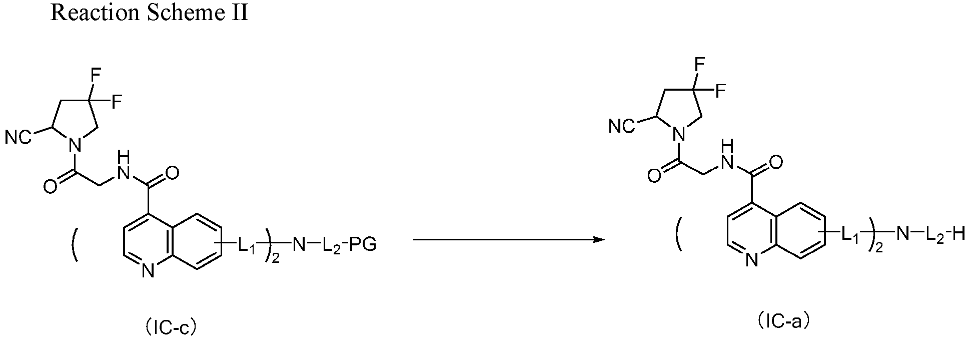

The compound of formual (IC-c) is converted into the compound of formula (IC-a) under acidic or basic conditions or in the presence of hydrogen/catalyst(e.g. palladium/carbon), wherein PG represents a common amino protecting group, such as tert-butyloxycarbonyl(Boc), benzyloxycarbonyl(Cbz), 9-fluorenylmethyloxycarbonyl(Fmoc), allyloxycarbonyl(Alloc), para-methoxylbenzyl(PMB), triphenylmethyl(Trt), etc., or a hydroxyl protecting group, such as methyl, ethyl, benzyl, methanesulfonyl, p-toluenesulfonyl, or a silicon-based protecting group, etc., wherein variables of L₁, L₂, and the bifunctional chelator are as defined herein.

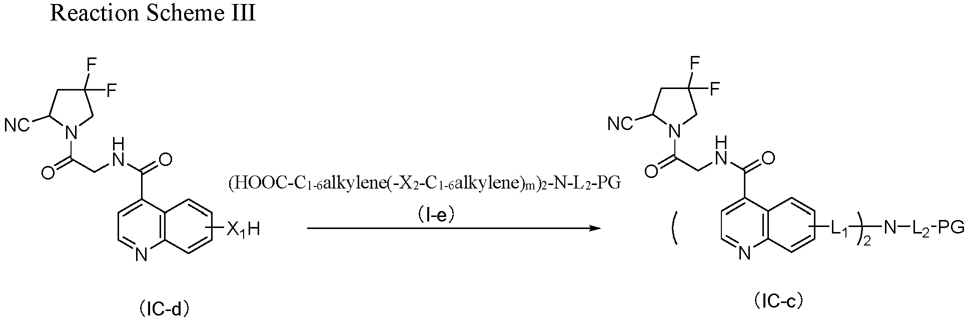

The compound of formula (IC-d) is condensed with the compound of formula (I-e) under suitable conditions. For example, to a mixture of the compounds of formula (IC-d) and formula (I-e) in an inert solvent (e.g. dimethyl sulfoxide, N,N-dimethyl formamide, acetonitrile, tetrahydrofuran, methylene chloride) are added a condensing agent (e.g. 2-(7-aza-1H-benzotriazol-1-yl) -1,1,3,3-tetramethyluronium hexafluorophosphate (HATU), O-benzotriazol-N,N,N',N'-tetramethyluronium hexafluorophosphate (HBTU), dicyclohexylcarbodiimide(DCC), N,N'-diisopropylcarbodiimide(DIC), 1-(3-dimethylaminopropyl)-3-ethylcarbodiimide(EDCI), ethyl cyanoglyoxylate-2-oxime), and a base (e.g. N-methylmorpholine, 4-dimethylaminopyridine, triethylamine, diisopropylethylamine, etc) for reaction to give the compound of formula (IC-c), wherein, variables of L₁, L₂, and the bifunctional chelator are as defined herein.

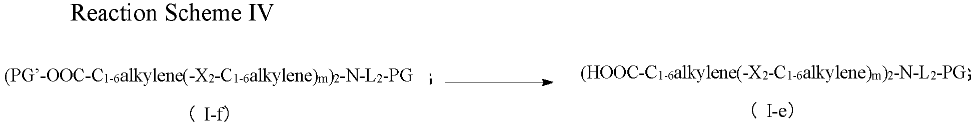

The protecting group PG' is removed from the compound of formula (I-f) under suitable conditions to give the compound of formula (I-e).

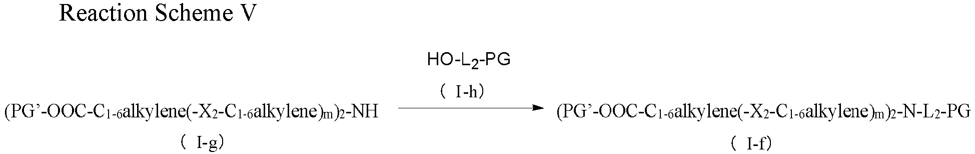

The compound of formula (I-g) is condensed with the compound of formula (I-h) under suitable conditions. For example, to a mixture of the compounds of formula (I-g) and formula (I-h) in an inert solvent (e.g. dimethyl sulfoxide, N,N-dimethyl formamide, acetonitrile, tetrahydrofuran, methylene chloride) are added a condensing agent (e.g. 2-(7-aza-1H-benzotriazol-1-yl)-1,1,3,3-tetramethyluronium hexafluorophosphate (HATU), O-benzotriazol-N,N,N',N'-tetramethyluronium hexafluorophosphate (HBTU), dicyclohexylcarbodiimide(DCC), N,N'-diisopropylcarbodiimide(DIC), 1-(3-dimethylaminopropyl)-3-ethylcarbodiimide(EDCI), ethyl cyanoglyoxylate-2-oxime), and a base (e.g. N-methylmorpholine, 4-dimethylaminopyridine, triethylamine, diisopropylethylamine, etc) for reaction to give the compound of formula (I-f).

### EXAMPLES

The following examples are provided to illustrate the preparation of the compounds of the invention, and do not limit the present disclosure in any way.

### I. Experiment Reagents and Instruments

### 1. Experiment Reagents

Unless specified to the contrary, the starting material and reagents are commercially available, or prepared by known methods.

### 2. Experiment instruments and high performance liquid chromatography (HPLC) Method

HPLC instrument Agilent 1260 HPLC system (equipped with a diode array detector) was purchased from Agilent, USA, HPLC radioactive signal detector (Gabi star γ-ray test) was purchased from Raytest, Germany. Semi-preparative C18 reversed phase column (ReproSil-Pur Basic C18, 5 um, 250×10.0 mm), analytic C18 reversed phase column (ReproSil-Pur Basic C18, 5 um, 250×4.6 mm) were purchased from Dr.Maisch, Germany. Lyophilizer (FD-1D-50) was purchased from Beijing Biocool Experiment Instrument Co. Ltd. Radioactive γ-counter (Wizard-2470) was purchased from PerkinElmer, USA. Dose calibration (CRC-25R) was purchased from Capintec, USA.

Method 1 for analysis and purification of the target product by HPLC: Agilent 1260 HPLC system equipped with analytic or semi-preparative C18 reversed chromatography column, the flow rate was set as 1 mL/min for analysis, and 4 mL/min for semi-preparation. Gradient elution was continued for 25 min, wherein mobile phase A was deionized water (containing 0.05% TFA), mobile phase B was acetonitrile (containing 0.05% TFA). The gradient elution is set as 85% A and 15% B at the starting point, 85%A and 15% B at 5 min, 20% A and 80% B at 20 min, 85% A and 15% B at 25 min.

Method 2 for analysis and purification of the target product by HPLC: the instruments and flow rate were the same as those of method 1. The gradient elution was continued for 20 min, wherein mobile phase A was deionized water (containing 0.05% TFA), mobile phase B was acetonitrile (containing 0.05% TFA). The gradient elution was set as 95% A and 5% B at the starting point, 95%A and 5% B at 5 min, 70% A and 30% B at 10 min, 70% A and 30% B at 15 min, 95% A and 5% B at 15.1 min, 95% A and 5% B at 20 min.

Method 3 for analysis and purification of the target product by HPLC: the instruments and flow rate were the same as those of method 1. Gradient elution was continued for 20 min, wherein mobile phase A was deionized water (containing 0.05% TFA), mobile phase B was acetonitrile (containing 0.05% TFA). The gradient elution was set as 95% A and 5% B at the starting point, 55%A and 45% B at 14 min, 95% A and 5% B at 20 min.

### II. Experiment Method and Results

The abbreviations used in the following examples have the following meaning respectively.

| | |
|---|---|
| DIPEA | diisopropylethylamine |
| DMF | N,N-dimethylformamide |
| EP tube | Eppendorf tube |
| Fmoc | 9-fluorenylmethoxycarbonyl |
| HATU | 2-(7-aza-1H-benzotriazol-1-yl)-1,1,3,3-tetramethyluronium hexafluorophosphate |
| HPLC | High performance liquid chromatography |
| NHS -HYNIC | (6-(2-(2- sulfobenzylidene) hydrazinyl) nicotinic acid N-hydroxysuccinimide ester) |
| NHS-DOTA | 1,4,7,10-tetraazacyclododecane-1,4,7,10-tetraacetic acid N-hydroxysuccinimide monoester |
| MBq | Million becquerel |
| mCi | millicurie |
| mg | milligram |
| min | minute |
| mL | milliliter |
| mmol | millimole |
| PEG | polyethylene glycol |
| tBu | Tert-butyl |
| TLC | Thin layer chromatography |
| TOF MS | Time of flight mass spectrography |
| Tricine | Tris(hydroxymethyl)methylglycine |
| TPPTS | Trisodium triphenylphosphine-3,3',3"-trisulfonate |
| µL | microliter |
| µg | microgram |
| µm | micrometer |
| µmol | micromole |

### Example 1 Synthesis of the intermediate compound Fmoc-NH-PEG₄-CO-N-bis(PEG₄-carboxylic acid) (compound 4)

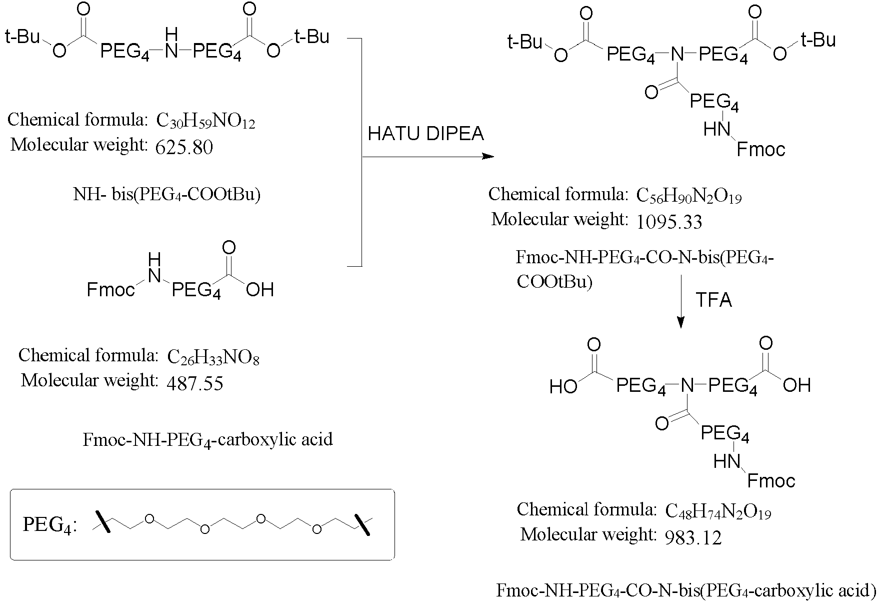

### 1. Synthesis of Fmoc-NH-PEG₄-CO-N-bis(PEG₄-COOtBu)

To a 50ml reaction flask were added 10.00g of NH-bis(PEG₄-COOtBu), 7.79g of Fmoc-NH-PEG₄-carboxylic acid, 6.08g of HATU, 40ml of DMF, which were stirred and dissolved at 20-30°C. Then, DIPEA was added dropwise, which was accompanied with significant exothermic phenomenon. The temperature rose up to about 50°C. Upon the completion of addition, the reaction mixture was stirred at 20-30°C for 2.0 hrs. TLC(methylene chloride:methanol = 10:1, one drop of ammonium hydroxide was added) detection showed that the reaction was completed. To the reaction system was added 200ml of water, and then extracted with methylene chloride(30ml×4). The combined organic phase was dried over anhydrous sodium sulfate, and filtered. The filtrate was concentrated under reduced pressure to dryness, and then purified by silica column chromatography (eluent: methylene chloride to methylene chloride/methanol (10/1)) to give 17.5g of the product, yielding 100%.

### 2. Synthesis of Fmoc-NH-PEG₄-CO-N-bis(PEG₄-carboxylic acid) (compound 4)

To a 50ml flask were added 17.50 g of Fmoc-NH-PEG₄-CO-N-bis(PEG₄-COOtBu), 14ml of trifluoroacetic acid, and 7.0ml of methylene chloride, which were stirred at 20-30°C for 20-22 hrs. TLC (methylene chloride: methanol = 10:1, one drop of acetic acid was added) detection showed that the reaction was completed. To the reaction system was added 200ml of water, and then extracted with methylene chloride (30ml×4). The combined organic phase was concentrated under reduced pressure to dryness, and then was purified with preparative HPLC (eluent: water-acetonitrile). The fraction was concentrated under reduced pressure to give 14.14 g of the product, yielding 90%.

### Example 2 Synthesis of the compound HYNIC-PEG₄-CO-N-bis(PEG₄-7N) (hereinafter referred to as H7ND)

### Step 1: Synthesis of (S)-7-amino-N-(2-(2-cyano-4,4-difluoropyrrolidin-1-yl)-2-oxoethyl)quinolin-4-carboxyamide (compound 3, hereinafter referred to as 7N)

84 mg of (S)-1-(2-aminoacetyl)-4,4-difluoropyrrolidin-2-carbonitrile hydrochloride (compound 1) (444 µmol, 1.1 eq) was dissolved in 1000 µL of ultra dried DMF, 76 mg of 7-aminoquinolin-4-carboxylic acid (compound 2) (404 µmol, 1.0 eq) was dissolved in 2000 µL of ultra dried DMF, the solution of compound 1 in DMF and the solution of compound 2 in DMF were combined in a 10mL reaction tube, to which was then added 1315 µL of HATU (dissolved in ultra dried DMF, 0.3 µmol/µL) (404 µmol, 1.0 eq). To the mixed solution was added 320 µL of DIEA (1616 µmol, 4.0 eq) in two portions with an interval of 0.5 h at room temperature. The reaction was shaken slightly for 1 hr. The reaction was monitored by HPLC (method 1). After the reaction was completed, the target product was isolated and purified (method 2). The fraction corresponding to the elution peak of 14.8 min in semi-preparative HPLC was collected, combined and freeze-dried by vacuum freeze-drying method to give 131 mg of the product 7N (compound 3). A small amount of the product was dissolved and identified by HPLC to have purity of 99.2%. ESI mass spectrography confirmed that it was the desired product, ESI-MS: m/z=360.13 [M+H]⁺, theoretical molecular weight: 359.12_{∘}

### Step 2: Synthesis of Fmoc-NH-PEG₄-CO-N-bis(PEG₄-7N) (compound 5)

22.5mg of 7N (compound 3) (62.66 µmol, 3.0 eq) was dissolved in 500 µL of ultra dried DMF. 26.8 mg of Fmoc-NH-PEG₄-CO-N-bis(PEG₄-carboxylic acid) (compound 4) (27.2 µmol, 1.3eq) was dissolved in 200 µL of ultra dried DMF. The solution of compound 3 in DMF and the solution of compound 4 in DMF were combined in a 1.5 mL EP tube, to which was then added 142 µL of HATU (dissolved in ultra dried DMF, 0.59 µmol/µL) (83.8 µmol, 4.0 eq). To the mixed solution was added 27.8 µL of DIPEA (166.8 µmol, 8.0 eq)in one portion. The reaction was shaken for 48 hrs at room temperature. The reaction was monitored by HPLC (method 1). After the reaction was completed, the target product was isolated and purified (method 3). The fraction corresponding to the elution peak of 11.8 min in semi-preparative HPLC was collected, combined, and freeze-dried by vacuum freeze-drying method to give 14.8 mg of saffron solid (compound 5). A small amount of the product was dissolved and identified by HPLC to have purity of 98.1%. MALDI-TOF mass spectrography confirmed that it was the desired product, MALDI-TOF-MS: m/z=1665.77 [M+H]⁺, 1687.75 [M+Na]⁺, 1703.73 [M+K]⁺, theoretical molecular weight: 1664.71_{∘}

### Step 3: Synthesis of NH₂-PEG₄-CO-N-bis(PEG₄-7N) (compound 6)

12.8 mg of Fmoc-NH-PEG₄-CO-N-bis(PEG₄-7N) (compound 5) was dissolved in 200 µL of acetonitrile. Upon the addition of 50 µL of piperidine, the reaction was continued for 20 min at room temperature. The reaction process was monitored by HPLC (method 3) . After the reaction was completed, the target product was isolated and purified (method 3). The fraction corresponding to the elution peak of 8.4 min in semi-preparative HPLC was collected, combined, and freeze-dried by vacuum freeze-drying method to give 9.2 mg of yellow solid (compound 6). A small amount of product was dissolved, and identified by HPLC to have purity of 99.5%.

### Step 4: Synthesis of HYNIC-PEG₄-CO-N-bis(PEG₄-7N) (compound 8, H7ND)

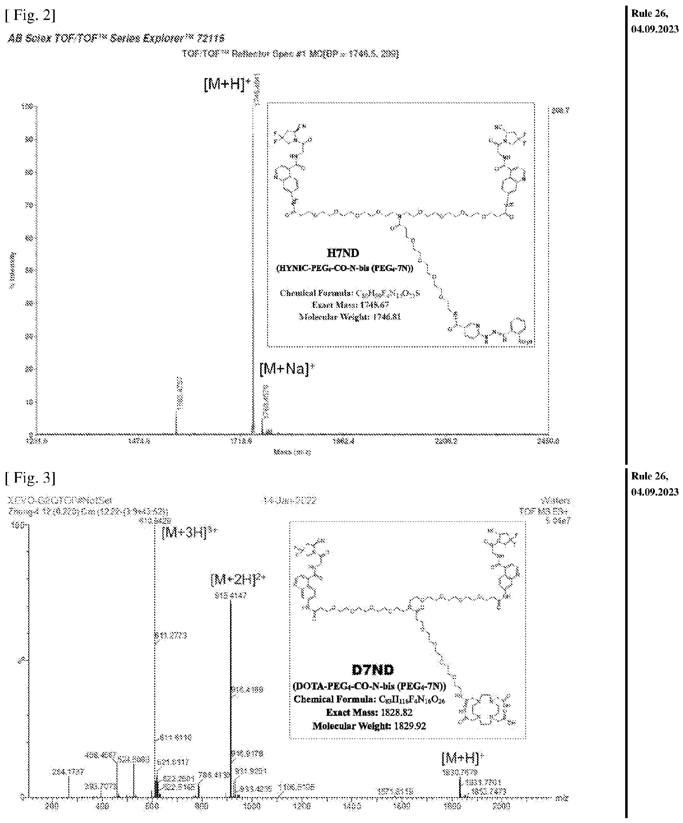

8.0 mg of NH₂-PEG₄-CO-N-bis(PEG₄-7N) (compound 6) was dissolved in 200 µL of ultra dried DMF. 6.0 mg of NHS-HYNIC (compound 7) was dissolved in 200 µL of ultra dried DMF. The solution of compound 6 in DMF and the solution of compound 7 in DMF were combined in a 1.5 mL EP tube, to which was then added 10 µL of DIPEA. The reaction was shaken for 2 hrs at 30°C. The reaction process was monitored by HPLC (method 3). After the reaction was completed, the target product was isolated and purified (method 3). The fraction corresponding to the elution peak of 9.5 min in semi-preparative HPLC was collected, combined, and freeze-dried by vacuum freeze-drying method to give 6.5 mg of pale yellow solid (compound 8, H7ND). A small amount of product was dissolved, and identified by HPLC to have purity of 99.2%. MALDI-TOF mass spectrography confirmed that it was the desired product, MALDI-TOF-MS: m/z=1746.48 [M+H]⁺, 1768.46 [M+Na]⁺, theoretical molecular weight: 1746.81 (mass spectrography analysis results are as shown is Figure 2) .

### Example 3 Synthesis of the compound DOTA-PEG₄-CO-N-bis(PEG₄-7N) (hereinafter referred to as D7ND)

NH₂-PEG₄-CO-N-bis(PEG₄-7N) (compound 6) was obtained by reference to step 1 to step 3 of Example 2.

6.0 mg of NH₂-PEG₄-CO-N-bis(PEG₄-7N) (compound 6) was dissolved in 200 µL of ultra dried DMF. 6.0 mg of NHS-DOTA was dissolved in 200 µL of ultra dried DMF. The solutions of the two compounds were combined in a 1.5 mL EP tube, to which was then added 10 µL of DIPEA. The reaction was shaken for 2 hrs at 30°C. The reaction process was monitored by HPLC (method 3). After the reaction was completed, the target product was isolated and purified (method 3). The fraction corresponding to the elution peak of 8.4 min in semi-preparative HPLC was collected, combined, and freeze-dried by vacuum freeze-drying method to give 8.0 mg of pale yellow solid (D7ND). A small amount of product was dissolved, and identified by HPLC to have purity of 99.4%. TOF MS (ES+) mass spectrography confirmed that it was the desired product, m/z=1830.77 [M+H]⁺, 915.41 [M+2H]²⁺, 610.94 [M+3H]³⁺, theoretical molecular weight: 1829.92 (mass spectrography analysis results are as shown in Figure 3) .

### Example 4 Preparation of H7ND freeze-dried kit

1 mg of H7ND was dissolved in 1 mL of 35% ethanol/water with the concentration of 1 µg/µL;then 1 mL of a mixed solution containing 40 µg of H7ND(40 µL), 2.0 mg of Tricine, 3.0 mg of TPPTS, 29.55 mg of succinic acid, and 17.0 mg of sodium hydroxide was prepared, and then filtered through 0.22 µm filter membrane, and added into a sterile vial of 10 mL. The mixed solution was freeze-dried, and then capped to give a H7ND freeze-dried kit for ^{99m}Tc labeling.

### Example 5 Preparation and quality control analysis of ^{99m}Tc-H7ND

1 mg of H7ND was dissolved in 1 mL of 35% ethanol/water with the concentration of 1 µg/µL; then 1 mL of a mixed solution containing 40 µg of H7ND (40 µL), 2.0 mg of Tricine, 3.0 mg of TPPTS, 29.55 mg of succinic acid, and 17.0 mg of sodium hydroxide was prepared, and then filtered through 0.22 µm filter membrane, and added into an aceptic vial of 10 mL. The mixed solution was freeze-dried, and then capped to give a freeze-dried kit for ^{99m}Tc labeling.

20~35mCi (740~1300 MBq) of sodium pertechnetate[^{99m}Tc] injection was taken for use. A H7ND freeze-dried kit for injection checked for no damage was reserved within the shelf life for use. Under aseptic operation conditions, 20~35 mCi (740~1300 MBq) of sodium pertechnetate[^{99m}Tc] injection was extracted into a sterile syringe, and then normal saline injection was added to 1 mL. It was injected into the H7ND freeze-dried kit, and then sufficiently shaken so that the solid in the kit was completely dissolved. The kit bottle was heated in a water bath at 100 °C for 20 min, and was then taken out of the water bath and cooled to room temperature to give ^{99m}Tc-H7ND injection.

After ^{99m}Tc-H7ND labeling was completed, one drop was taken by a sterile syringe and applied to flash silica gel thin-layer paper chromatography (ITLC-SG) for quality control analysis: the fast thin layer chromatography paper was used as the support, and two kinds of elution systems of acetone, and acetonitrile/normal saline mixture system (V_{acetonitrile}/V_{normal saline}=1/1) were used to elute the sample. After drying the chromatography paper in the air, the data were collected by Bioscan radioactivity thin layer scanner. The images, radioactivity percentages and Rf values were obtained to calculate the radiochemical purity of drugs after the data were collected. The main radioactive substances contained in the labeling solution were technetium labeled compound (^{99m}Tc-H7ND), free technetium (^{99m}TcO₄⁻) and colloidal technetium (^{99m}TcO₂). The Rf values obtained for each radioactive component in the two elution systems are shown in Table 1.

**Table 1 Rf value of each radioactive component in ^{99m}Tc-H7ND labeling system**

| Elution system | Rf value of label | Rf value of ^{99m}TcO₄ | Rf value of ^{99m}TcO₂ |
|---|---|---|---|
| acetone | 0-0.1 | 0.9-1.0 | 0-0.1 |
| Acenitrile/normal saline 1:1 | 0.9-1.0 | 0.9-1.0 | 0-0.1 |

Quality control analysis of 99^{m}Tc-H7ND may also be completed via Radio-HPLC method. HPLC (equipped with Raytest Gabistar radioactivity detector and Agilent-35900E digital to analog converter) and C18 reversed phase column (ReproSil-Pur Basic C18, 5 um, 250×4.6 mm) were used, and gradient elution was continued for 25 min with the flow rate of 1 mL/min, wherein mobile phase A was 0.01M PBS buffer of pH 7.4, mobile phase B was acetonitrile. Elution gradient was set as 90% A and 10% B at the starting point, 60% A and 40% B at 20 min, 90% A and 10% B at 25 min. The retention time of ^{99m}Tc-H7ND was 16.1 min, and that of ^{99m}TcO₄⁻ was 3.5 min.

### Example 6 Preparation and purification of ⁶⁸ Ga-D7ND

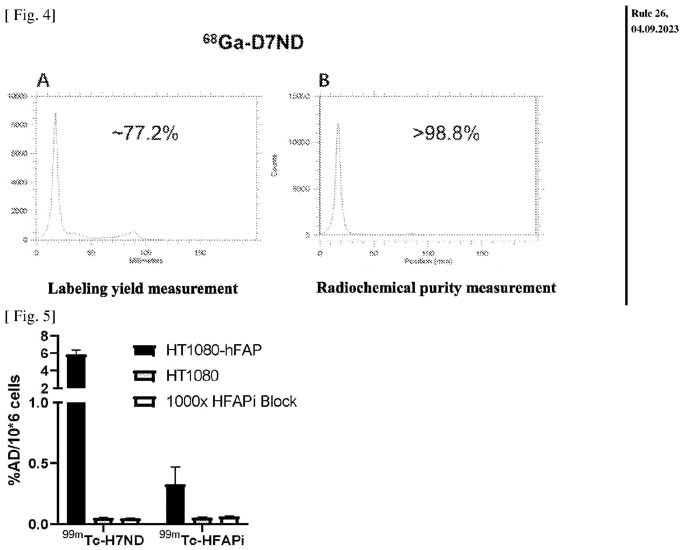

1 mg of D7ND was dissolved in 1 mL of pure water to prepare a solution with the concentration of 1 µg/µL for use; Sep-Pak C18 cartridge was flushed with 5 mL of anhydrous ethanol and 10 mL of water for injection in sequence, and was then dried over 10 mL of air to complete activation. To 20 µL of D7ND solution (containing 20 µg of D7ND) in 1.5 mL EP tube were added 97 µL of 1M sodium acetate, and 1.0 mL of ⁶⁸GaCl₃ eluent (5-15 mCi, 0.05 M hydrochloric acid medium) in sequence. The mixture was reacted in an incubator at 90°C for 15 min. Radio-iTLC analysis showed that the labeling rate was 77% (as shown in Figure 4A) . After the reaction was completed, the reaction liquid was extracted with a 5 mL injection syringe filled with 2 mL of water for injection pumped in advance, and was loaded on the activated C18 cartridge. The C18 cartridge was washed with 10 mL of water for injection and the waste liquid was discarded. Finally, the C18 cartridge was eluted with 0.5 mL of 80% ethanol and the labeled compound was collected into an aseptic vial. The labeled compound was diluted with 2 mL of normal saline and filtered through 0.22µm microfiltration membrane, and then was reserved in an aseptic vacuum flask for use. The radiochemical purity of ⁶⁸Ga-D7ND was greater than 98% as determined by Radio-iTLC analysis (as shown in Figure 4B).

### Example 7 Preparation and purification of ¹⁷⁷Lu-D7ND

1 mg of D7ND was dissolved in 1 mL pure water to prepare a solution with the concentration of 1 µg/µL for use; Sep-Pak C18 cartridge was flushed with 5 mL of anhydrous ethanol and 10 mL of water for injection in sequence, and was then dried over 10 mL of air to complete activation. To 40 µL D7ND solution (containing 40 µg of D7ND) in 1.5 mL EP tube were added 0.2 mL of 4M sodium acetate buffer solution (pH 4.5), and 0.1 mL of ¹⁷⁷LuCl₃ solution (10-50 mCi, 0.01 M hydrochloric acid medium) in sequence. The mixture was reacted in an incubator at 95°C for 20 min. After the reaction was completed, the reaction liquid was extracted with a 5 mL injection syringe filled with 2 mL of water for injection in advance, and was loaded on the activated C18 cartridge. The C18 cartridge was flushed with 10 mL of water for injection and the waste liquid was discarded. Finally, the C18 cartridge was eluted with 0.5 mL of 80% ethanol and the labeled compound was collected into an aseptic vial. The labeled compound was diluted with 2 mL normal saline and filtered through 0.22µm microfiltration membrane, and then was reserved in an aseptic vacuum flask for use. The radiochemical purity of ¹⁷⁷Lu-D7ND was greater than 95% as determined by Radio-HPLC analysis.

### Example 8 Preparation and purification of ⁹⁰Y-D7ND

1 mg of D7ND was dissolved in 1 mL pure water to prepare a solution with the concentration of 1 µg/µL for use; Sep-Pak C18 cartridge was eluted with 5 mL of anhydrous ethanol and 10 mL of water for injection in sequence, and was then dried over 10 mL of air to complete activation. To 40 µL D7ND solution (containing 40 µg of D7ND) in 1.5 mL EP tube were added 0.2 mL of 4M sodium acetate buffer solution (pH 4.5), and 0.1 mL of ⁹⁰YCl₃ solution (10-50 mCi, 0.01 M hydrochloric acid medium) in sequence. The mixture was reacted in an incubator at 95°C for 20 min. After the reaction was completed, the reaction liquid was extracted with a 5 mL injection syringe filled with 2 mL of water for injection in advance, and was loaded on the activated C18 cartridge. The C18 cartridge was flushed with 10 mL of water for injection and the waste liquid was discarded. Finally, the C18 cartridge was eluted with 0.5 mL of 80% ethanol and the labeled compound was collected into an aseptic vial. The labeled compound was diluted with 2 mL of normal saline and filtered through 0.22µm microfiltration membrane, and then was reserved in an aseptic vacuum flask for use. Radioactivity of the final product was measured and the yield was calculated to be about 75%. The radiochemical purity of ⁹⁰Y-D7ND was greater than 99% as determined by Radio-HPLC analysis.

### Example 9 Preparation and purification of Al¹⁸F-N7ND

1 mg of D7ND and 1 mg of AlCl₃ were respectively dissolved in 1 mL of a solution containing 50 mM potassium biphthalate (pH 4.0) and 100mg/mL trehalose to prepare a solution with the concentration of 1 µg/µL for use; Sep-Pak C18 cartridge was flushed with 5 mL of anhydrous ethanol and 10 mL of water for injection in sequence, and was then dried over 10 mL of air to complete activation. To 100 Ml of ¹⁸F⁻ solution (~ 3.2 GBq) in a 1.5 mL EP tube was added 3 µL of AlCl₃ solution (containing 3 µg AlCl₃), and the reaction was continued for 5 min at room temperature. Then, 40 µL N7ND solution (containing 40 µg N7ND) was added and the reaction was continued for 15 min at 90°C. After the reaction was completed, the reaction liquid was extracted with a 5 mL injection syringe filled with 2 mL of water for injection in advance, and was loaded on the activated C18 cartridge. The C18 cartridge was flushed with 10 mL of water for injection and the waste liquid was discarded. Finally, the C18 cartridge was eluted with 0.5 mL of 80% ethanol and the labeled compound was collected into an aseptic vial. The labeled compound was diluted with 2 mL of normal saline and filtered through 0.22µm microfiltration membrane, and then was reserved in an aseptic vacuum flask for use. Radioactivity of the final product was determined and the yield was calculated to be about 27%. The radiochemical purity of Al¹⁸F-N7ND was greater than 95% as determined by Radio-HPLC analysis.

### Example 10 Biological Experiment

### Experimental Cells and Animals

Female BALB/c nude mice of SPF grade, 4~5 weeks old, and female Kunming mice of SPF grade, 6 weeks old which were purchased from Beijing Vital River Laboratory Animal Technology Co., Ltd. HT-1080 human fibrosarcoma cells (FAP negative), HT-1080-hFAP human fibrosarcoma cells (cell lines stably transfected with hFAP overexpression), and U87MG human glioma cells (FAP was highly expressed in tumor tissues after oncogenesis) were cultured in DMEM medium containing 10% fetal bovine serum (FBS). The cells were subcultured routinely in an incubator containing 5% CO₂ at 37°C. 5×10⁶ U87MG cells were inoculated subcutaneously into the right front flanks of BALB/c nude mice of 4 ~ 5 weeks old, and the mice were fed under SPF condition. The mice were used for imaging and biodistribution experiment when the tumor size reached 100~300 mm³.

### 1, In vitro cell binding assay of ^{99m}Tc-H7ND

In order to verify ^{99m}Tc-H7ND of dimeric form has higher affinity for FAP-α than ^{99m}Tc-HFAPi based on monomeric FAP-α (the compound of the patent CN111991570B), we respectively prepared and used ^{99m}Tc-H7ND and ^{99m}Tc-HFAPi of the same specific radioactivity to perform in vitro cell binding assay.

In the in vitro cell binding assay, the HT-1080-hFAP human fibrosarcoma cell stably transfected with hFAP was used as positive control cell, HT-1080 human fibrosarcoma cell (FAP negative) was used as negative control cell. In vitro cell binding assay was conducted as follows: the culture solutions of the HT-1080-hFAP human fibrosarcoma cell and the HT-1080 human fibrosarcoma cell were respectively transferred into 15 mL of centrifuge tubes, centrifuged at a force of 150 ×g for 5 min, and the supernatant was discarded. 10 mL of PBS was added and the cells were resuspended and washed. After centrifugation and discarding the supernatuant, the cells were resuspended again by PBS, and counted respectively through cell counting boards. Then, the cells were collected into 1.5 mL EP tubes at 2×10⁶ cells/tube, and blocked by 2% BSA solution for 0.5 hr at room temperature. Labeled ^{99m}Tc-H7ND and ^{99m}Tc-HFAPi were respectively added into EP tubes containing cells, 11 kBq/tube, mixed gently, and incubated in ice bath for 20 min. For the study of blocking the interaction between the probe and the cells, the HFAPi of 1,000× moles was added into the EP tube of the corresponding group for co-incubation. After the incubation was completed, the cells were resuspended by ice-cold PBS, centrifuged at a force of 150 ×g for 5 min, and the supernatant was discarded. Such washing was cycled for five times. Finally, the washed cells were lysed with 2 mol/L NaOH solution, and the cell lysate of each tube was collected and the radioactivity thereof was measured in γ counter. There were four parallel samples in every group of cell binding assay. The binding value of the cell and probe was expressed as the percentage (%AD) of radioactive counting per minute versus the added radiation dose after decay correction. The experimental results were shown in Figure 5. The binding between ^{99m}Tc-H7ND and the HT-1080-hFAP human fibrosarcoma cell had %AD value of 5.8% (±0.2%), the binding between ^{99m}Tc-HFAPi and the HT-1080-hFAP human fibrosarcoma cell had %AD value of 0.3% (±0.12%) . For the negative control group and blocking group, the binding values of ^{99m}Tc-H7ND and ^{99m}Tc-HFAPi were equivalent and significantly lower than the positive control group. The experimental results showed that both drugs could specifically bind to FAP-α protein at the same binding site, however, ^{99m}Tc-H7ND exhibited significantly higher binding affinity than ^{99m}Tc-HFAPi for about 18 times.

### 2, In vivo stability and metabolism study of ^{99m}Tc-H7ND in normal BALB/c mice

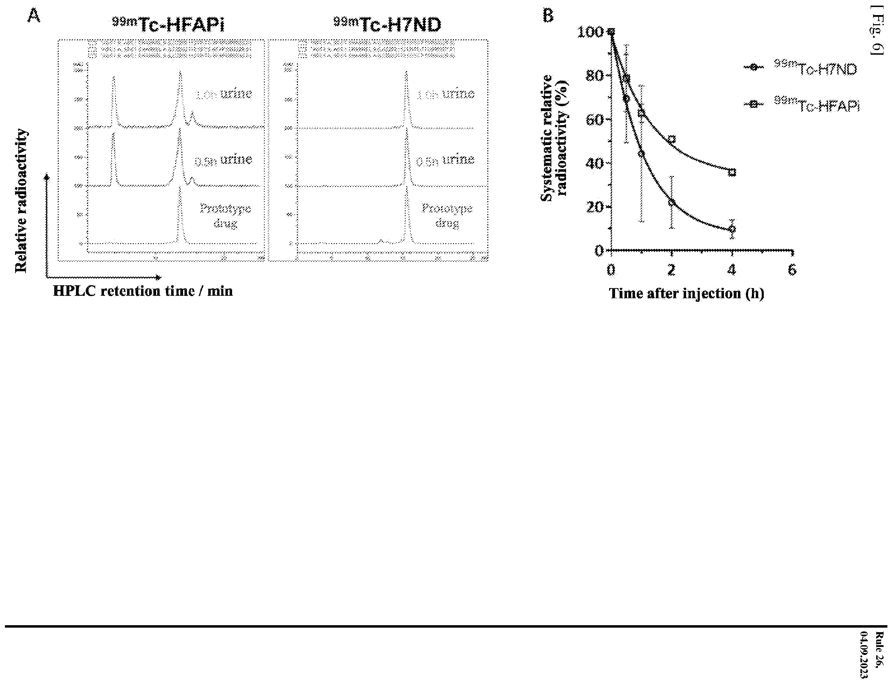

In order to explore ^{99m}Tc-H7ND of novel structure has more excellent in vivo pharmacokinetic property than ^{99m}Tc-HFAPi based on monomeric FAP-α, we divided BALB/c normal mice into two groups, three mice per group. Mice of the two groups was injected with 100 µL (~ 37 MBq) of ^{99m}Tc-H7ND and ^{99m}Tc-HFAPi respectively via trail vein, and at 0.5 hr and 1 hr after injection, the radiochemical purity of the drug in mice urine was analyzed with radioactive high performance liquid chromatography. Meanwhile, in the case that mice had normal activity, feeding, drinking, and excretion, systematic radioactivity was measured and recorded at 0, 0.5, 1, 2, and 4 hrs after injection. In vivo stability and metabolism experimental result was shown in Figure 6A. After metabolism in mice, ^{99m}Tc-HFAPi had a significant decomposition than ^{99m}Tc-H7ND, only about 50% of which could be excreted out of the body in the form of prototype drug at 1hr after injection, while ^{99m}Tc-H7ND of novel dimeric form maintained favorable in vivo stability during the period of observation without decomposition being observed. Figure 6B showed the difference of in vivo retention between the two drugs, there was less than 10% of ^{99m}Tc-H7ND maintained in the body at 4 hrs after injection, while there was about 35% of ^{99m}Tc-HFAPi maintained in the body at 4 hrs after injection, which undoubtedly increased unnecessary absorbed radiation dose of the patients as for radiopharmaceuticals. The experimental results showed that ^{99m}Tc-H7ND had significantly higher in vivo metabolism stability than ^{99m}Tc-HFAPi, and almost no decomposition phenomenon happened during the period of observation. However, nearly 50% of ^{99m}Tc-HFAPi based on monomeric FAP-α converted to metabolites. In addition, ^{99m}Tc-H7ND had significantly shorter in vivo retention time than ^{99m}Tc-HFAPi, which reduced radiation exposure of the patients and reduced the risk of adverse reaction caused by radiation to the patients.

### 3, SPECT/CT imaging of ^{99m}Tc-H7ND in U87MG tumor-bearing mice model

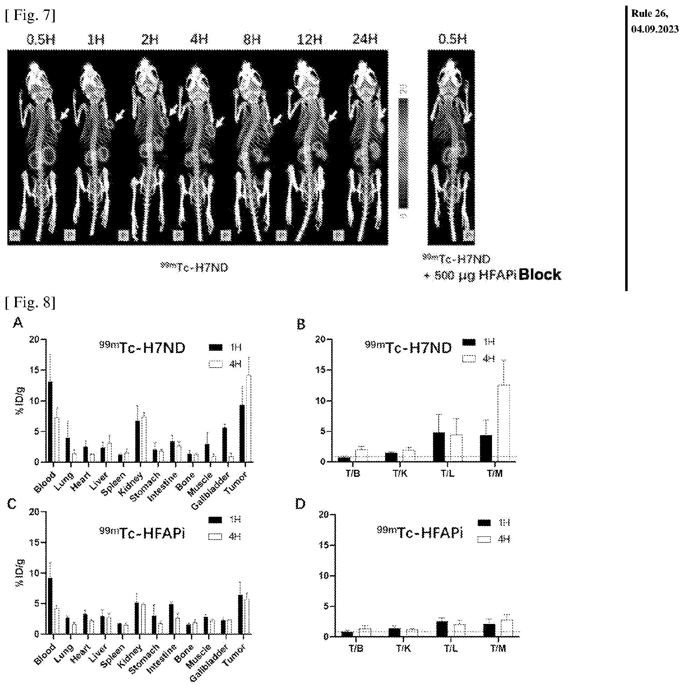

^{99m}Tc-H7ND was dissolved in normal saline at the concentrate of 37 MBq/100 µL. Each U87MG tumor-bearing mouse was injected with 100 µL (37 MBq) of the above solution via tail vein, and SPECT/CT imaging was performed at 0.5, 1, 2, 4, 8, 12, and 24 hrs after injection. Mice of the blocking group was injected with 100 µL (500 µg) HFAPi at the same time of injection of the above imaging drug ^{99m}Tc-H7ND, and imaged at 0.5 hr after injection. Mice were anesthetized by inhalation of 1.5% isoflurane-oxygen during imaging. SPECT imaging was reconstructed and fused with CT imaging to give 3D imaging (Posterior view) after imaging. Imaging results were shown in Figure 7, the tumor position was marked with arrows. The experimental results showed that ^{99m}Tc-H7ND could be taken up efficiently by tumor with high imaging contrast, which indicated that ^{99m}Tc-H7ND had excellent tumor specific targeting. In the experiment of blocking group, the tumor uptake of ^{99m}Tc-H7ND was significantly reduced without the tumor imaging being observed, which further demonstrated excellence of the tumor specific targeting of ^{99m}Tc-H7ND.

### 4. Biodistribution comparison of ^{99m}Tc-H7ND and ^{99m}Tc-HFAPi in U87MG tumor-bearing mice model

Sixteen U87UM tumor-bearing BALB/c nude mice (six weeks old) were randomly divided into four groups, four mice per group, wherein there were two groups for ^{99m}Tc-H7ND and ^{99m}Tc-HFAPi biodistribution experiments respectively. The prepared ^{99m}Tc-H7ND and ^{99m}Tc-HFAPi were dissolved in normal saline at the concentration of 370 kBq/100 µL. Each mouse was injected 100 µL (370 kBq) via tail vein. The mice were sacrificed at 1 and 4 hrs after injection, and the blood and major organs were taken out, weighed, and measured for radioactivity cpm count. Percentage injection dose per gram tissue (%ID/g) was calculated after decay correction. Biodistribution results were expressed as mean ± standard deviation (mean ± SD, n=4).

The experimental results were shown in Figure 8. Compared to ^{99m}Tc-HFAPi, ^{99m}Tc-H7ND had significantly higher tumor uptake absolute value, wherein tumor uptake of ^{99m}Tc-H7ND was 300% higher than that of ^{99m}Tc-HFAPi, and the uptake of ^{99m}Tc-H7ND by gall bladder and intestinal canal was 60% lower than that of ^{99m}Tc-HFAPi. Furthermore, metabolism and clearance of ^{99m}Tc-H7ND in normal organs were faster than those of ^{99m}Tc-HFAPi, which made the tumor/normal organ uptake ratio of ^{99m}Tc-H7ND was significantly higher than that of ^{99m}Tc-HFAPi which was beneficial to the nuclear medicine imaging in tumors. In particular, since SPECT imaging was very sensitive to background signal noise, higher tumor/normal organ uptake ratio was beneficial to diagnosis of minor tumor focus.

### 5. PET imaging comparison of ⁶⁸Ga-D7ND and ⁶⁸Ga-FAPi-04 in U87MG tumor-bearing mice model

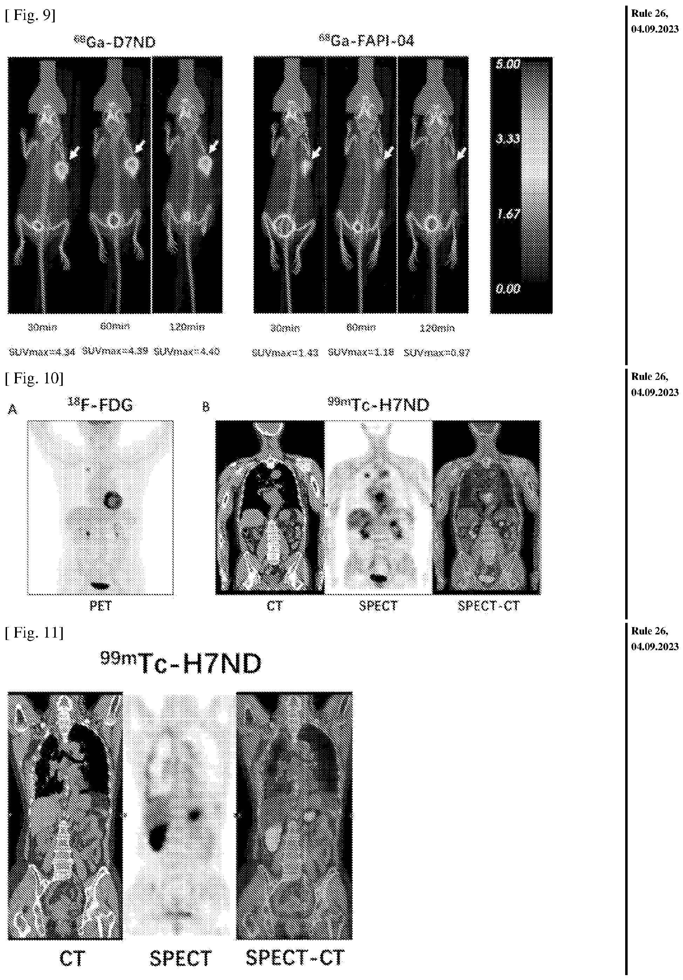

⁶⁸Ga-D7ND and ⁶⁸Ga-FAPi-04 were dissolved in normal saline at the concentrate of 18.5 MBq/100 µL respectively. Each U87MG tumor-bearing mouse was injected with 100 µL (18.5 MBq) of the above solution via tail vein, and Micro-PET/CT imaging was performed at 0.5, 1, and 2 hrs after injection. Mice were anesthetized by inhalation of 1.5% isoflurane-oxygen during imaging. PET imaging was reconstructed and fused with CT imaging to give 3D imaging(systematic MIP view) after imaging. Imaging results were shown in Figure 9, the tumor position was marked with arrows. The experimental results showed that ⁶⁸Ga-D7ND of the present invention had tumor SUVmax value of about more than 220% higher than ⁶⁸Ga-FAPi-04 based on monomeric FAPI-α, which indicated that ⁶⁸Ga-D7ND had significantly better tumor uptake, significantly higher imaging contrast, and higher tumor specificity than ⁶⁸Ga-FAPi-04 based on monomeric FAPI-α, which was beneficial to resolution of imaging and accuracy rate of diagnosis. In the context of diagnosis, the dose of ⁶⁸Ga-D7ND was significantly lower than that of ⁶⁸Ga-FAPi-04, which reduced the radiation exposure to the patients, and reduced the risk of adverse reaction caused by radiation to the patients.

### 6. ¹⁸F-FDG-PET and ^{99m}Tc-H7ND-SPECT imaging of lung tumor patients

A 71-year-old male patient was presented with cough for two months and pulmonary nodules for two weeks. Chest CT showed that there were irregular nodules in posterior segment of the right upper lobe apex, indicating a high possibility of peripheral lung cancer, and needle biopsy was recommended. Then, ¹⁸F-FDG-PET/CT imaging and ^{99m}Tc-H7ND-SPECT/CT imaging were performed respectively. ¹⁸F-FDG-PET/CT imaging result was shown in Figure 10A : a nodule shadow with a size of about 18.5mm×15.9mm×18.6mm was observed in the apical segment of the upper lobe of the right lung, lobulation, spinous processes and spiculation signs were observed, the adjacent bronchus was truncated, and the radionuclide uptake by the nodule was significantly increased, with SUVmax of 7.0. ^{99m}Tc-H7ND-SPECT/CT imaging was shown in Figure 10B: the nodule shadow in the upper lobe of the right lung with increased radionuclide distribution, the maximum T/N=4.13, was considered to be a malignant lung lesion. Finally, the intraoperative pathology confirmed invasive poorly differentiated adenocarcinoma in the upper lobe of the right lung. The results showed that ^{99m}Tc-H7ND-SPECT was not less effective than ¹⁸F-FDG-PET in detecting lung cancer in this case.

A 65-year-old female patient with lung adenocarcinoma received targeted therapy and immunotherapy after surgery, and the tumor markers increased progressively. Chest CT showed thoracic asymmetry, right thoracic collapse, diffuse thickening of the right pleura, and multiple nodules under the pleura. Enlarged lymph nodes with calcification were observed in the mediastinum, and there was a sign of small amount of pleural effusion on the right. Nuclear medicine imaging with ^{99m}Tc-H7ND-SPECT/CT was subsequently performed, and the imaging results were shown in Figure 11: the right pleura showed diffuse thickening, and locally scattered multiple nodular shadows with abnormal thickening of radionuclide distribution, the maximum T/N=4.58. Right hilar showed nodule shadows with abnormal thickening of radionuclide distribution, the maximum T/N=3.45. The lower right lobe and left lobe of the liver showed irregular slightly low-density foci with abnormal thickening of radionuclide distribution, the maximum T/N=2.83-3.51. The possibility of pleural metastasis, hilar lymph node metastasis and liver metastasis were considered. The final needle biopsy of pleural nodule confirmed the poorly differentiated adenocarcinoma invasion of fibrous tissue. The results showed that ^{99m}Tc-H7ND-SPECT/CT had excellent detection efficiency for pleural metastasis, lymph node metastasis, and liver metastasis of lung cancer in this case.

## Claims

1. A ligand compound of formula (IC) 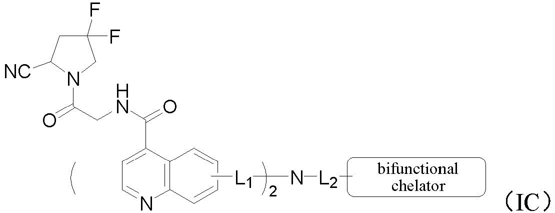 wherein,
L₁ is a functionalized linker of -X₁-CO-C₁₋₆ alkylene (-X₂-C₁₋₆ alkylene)ₘ-;
L₂ is a functionalized linker of -CO-C₁₋₆alkylene-(X₃-C₁₋₆alkylene-)ₙ-X₄-;
X₁, X₂, X₃, X₄ are each independently selected from the group consisting of O, S, NH, and NCH₃; the C₁₋₆alkylene is optionally substituted by halo, OH, NH₂, oxo, and/or cyano;
m, n are each an integer selected from 0, 1, 2, 3, 4, 5, 6, 7, 8, 9, or 10;
the bifunctional chelator is selected from the group consisting of 6-(2-(sulfobenzylidene)hydrazinyl)nicotinic acid (HYNIC), mercaptoacetyldiglycine(MAG₂), mercaptoacetyltriglycine(MAG₃), 1,4,7,10-tetraazacyclododecane-1,4,7,10-tetraacetic acid(DOTA), 1,4,7-triazacyclononane-1,4,7-triacetic acid(NOTA), 1,4,8,11-tetraazacyclotetradecane-1,4,8,11-tetraacetic acid (TETA), ethylenediaminetetraacetic acid (EDTA), diethylenetriamine-N,N,N',N',N''-pentaacetic acid (DTPA), 3,6,9,15-tetraazabicyclo[9.3.1]pentadecane-1(15),11,13-triene-3,6,9-triacetic acid (PCTA), RESCA, 1,4,7-triazacyclononane-1-glutaric acid-4,7-diacetic acid (NOTA-GA), 1,4,7,10-tetraazacyclododecane-1-glutaric acid-4,7,10-triacetic acid (DOTA-GA), 1,4,8,11-tetraazabicyclo[6,6,2]hexadecane-4,11-diacetic acid(CB-TE2A), 1,8-diamino-3,6,10,13,16,19-hexazabicyclo[6,6,6]eicosane(DiAmSar), 1-(4-isothiocyanatophenyl) -3-[6,17-dihydroxy-7,10,18,21-tetraoxo-27-(N-acetylhydroxylamino) -6,11,17,22-tetraazaheptacosane] (DFO) ;
or a pharmaceutically acceptable salt, stereoisomer thereof.

2. The ligand compound of claim 1, which is the compound of formula (IC-I), (IC-II), (IC-III), or (IC-IV) , 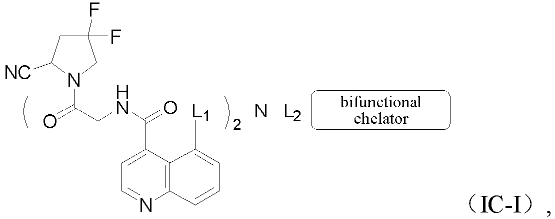 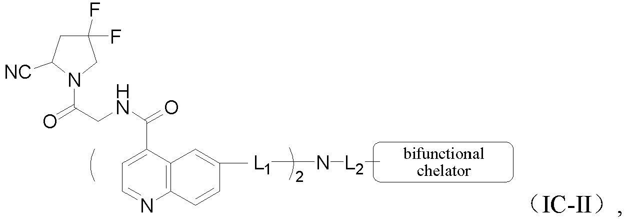 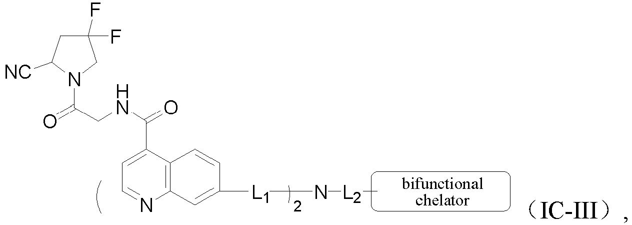 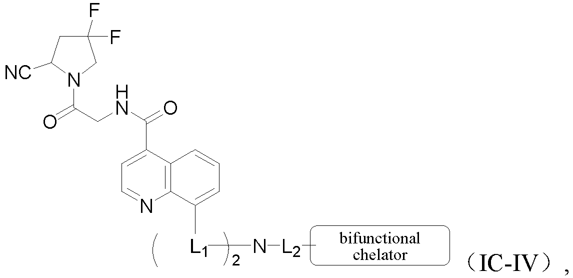
or a pharmaceutically acceptable salt, stereoisomer thereof;
wherein, L₁, L₂, and the bifunctional chelator are as defined in claim 1.

3. The ligand compound of claim 1, which is the compound of formula (ID), 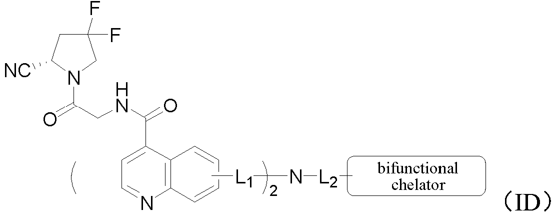
or a pharmaceutically acceptable salt, stereoisomer thereof;
wherein, L₁, L₂, and the bifunctional chelator are as defined in claim 1.

4. The ligand compound of claim 3, which is the compound of formula (ID-I), (ID-II), , or (ID-IV), 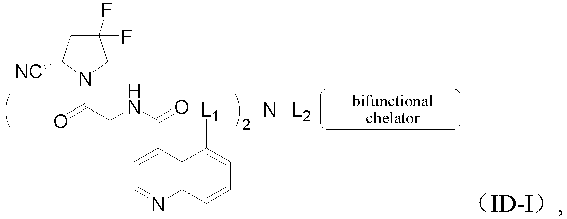 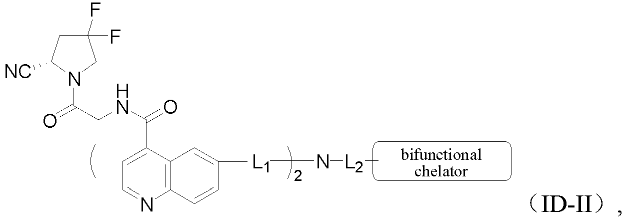 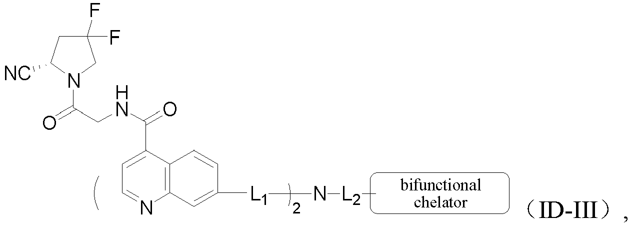 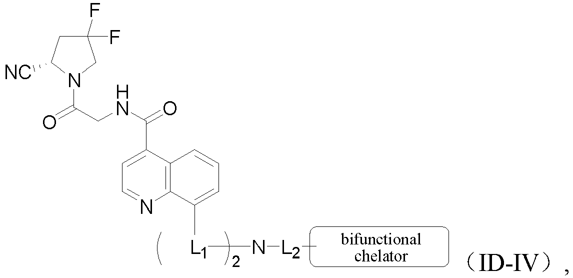
or a pharmaceutically acceptable salt, stereoisomer thereof;
wherein, L₁, L₂, the bifunctional chelator are as defined in claim 1.

5. The ligand compound of any of claims 1-4, wherein L₁ is the functionalized linker of -NHCO-C₁₋₆alkylene(-O-C₁₋₆alkylene)ₘ-, -NHCO-C₁₋₆alkylene(-NH-C₁₋₆alkylene)ₘ-, -NHCO-C₁₋₆alkylene(-NCH₃-C₁₋₆alkylene)ₘ-, -NCH₃-CO-C₁₋₆alkylene(-O-C₁₋₆alkylene)ₘ-, -NCH₃-CO-C₁₋₆alkylene(-NH-C₁₋₆alkylene)ₘ-, -NCH₃-CO-C₁₋₆alkylene(-NCH₃-C₁₋₆alkylene)ₘ-, -OCO-C₁₋₆alkylene(-O-C₁₋₆alkylene)ₘ-, -OCO-C₁₋₆alkylene(-NH-C₁₋₆alkylene)ₘ-, and/or -OCO-C₁₋₆alkylene(-NCH₃-C₁₋₆alkylene)ₘ-, wherein the alkylene is optionally substituted by halo, OH, NH₂, oxo, and/or cyano, m is an integer selected from 1, 2, 3, 4, 5, or 6;
L₂ is the functionalized linker of -CO-C₁₋₆alkylene-(NH-C₁₋₆alkylene-)ₙ-NH-, -CO-C₁₋₆alkylene-(NH-C₁₋₆alkylene-)ₙ-NCH₃-, -CO-C₁₋₆alkylene-(NH-C₁₋₆alkylene-)ₙ-O-, -CO-C₁₋₆alkylene-(O-C₁₋₆alkylene-)ₙ-NH-, -CO-C₁₋₆alkylene-(O-C₁₋₆alkylene-)ₙ-NCH₃-, -CO-C₁₋₆alkylene-(O-C₁₋₆alkylene-)ₙ-O-, -CO-C₁₋₆alkylene-(NCH₃-C₁₋₆alkylene-)ₙ-NH-, -CO-C₁₋₆alkylene-(NCH₃-C₁₋₆alkylene-)ₙ-NCH₃-, and/or -CO-C₁₋₆alkylene-(NCH₃-C₁₋₆alkylene-)ₙ-O-, wherein the alkylene is optionally substituted by halogen, OH, NH₂, oxo, and/or cyano, n is an integer of 1, 2, 3, 4, 5, or 6.

6. The ligand compound of claim 5, wherein L₁ is the functionalized linker of -NHCO-CH₂CH₂-(-O-CH₂CH₂)₄-; L₂ is the functionalized linker of -CO-CH₂CH₂-(O-CH₂CH₂-)₄-NH-.

7. The ligand compound of claim 1, which is selected from: 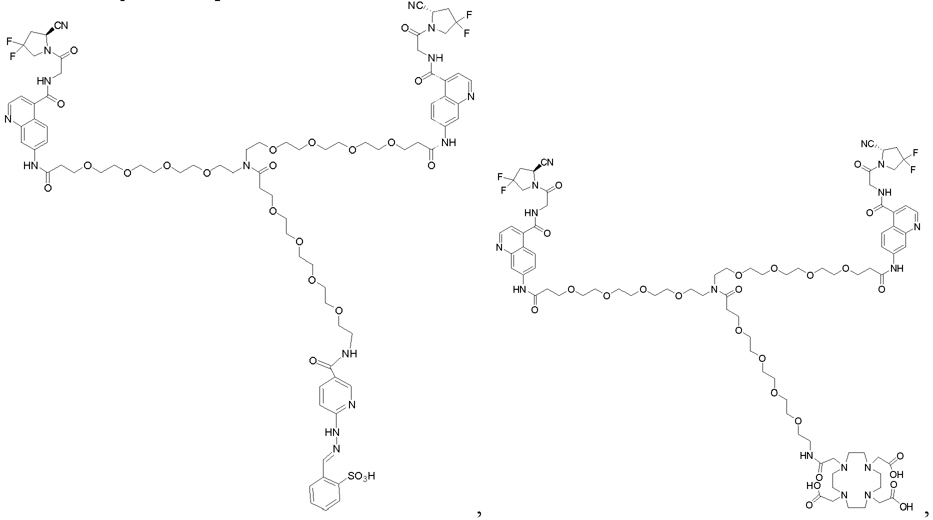 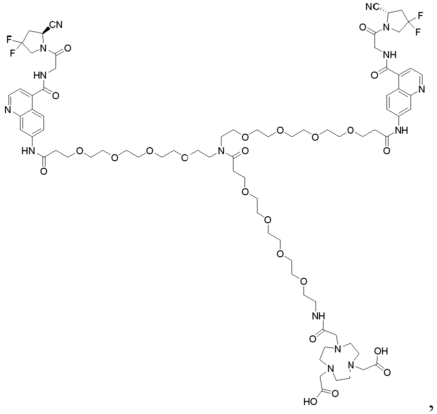 or a pharmaceutically acceptable salt, stereoisomer thereof.

8. A complex compound comprising the compound of any of claims 1-7 and a radionuclide, wherein the radionuclide is conjugated to the bifunctional chelator of the compound of any of claims 1-9 through a coordination bond, wherein the radionuclide is selected from the group consisting of alpha radiation emitting isotopes, beta radiation emitting isotopes, gamma radiation emitting isotopes, Auger electron emitting isotopes, X-ray emitting isotopes; preferably, the radionuclide is selected from the group consisting of ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁸Y, ⁹⁰Y, ¹⁴⁹Pm, ¹⁷⁷Lu, ⁴⁷Sc, ²¹²Bi, ²¹³Bi, ⁷²As, ¹²³I, ¹²⁴I, ¹³¹I, ²¹¹At, ²⁰¹Tl, ²¹²Pb, ⁶⁴Cu, ⁶⁷Cu, ¹⁹⁸Au, ²²⁵Ac, ²²³Ra, or ⁸⁹Sr.

9. The complex compound of claim 8, which is selected from: 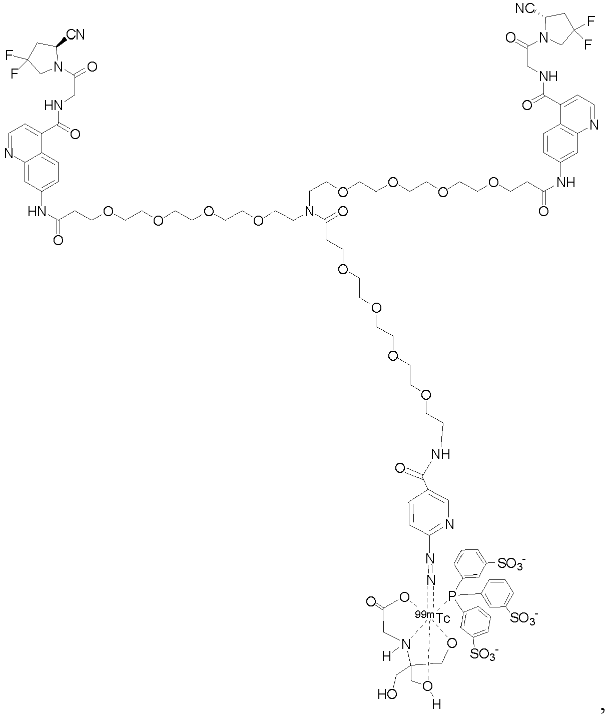 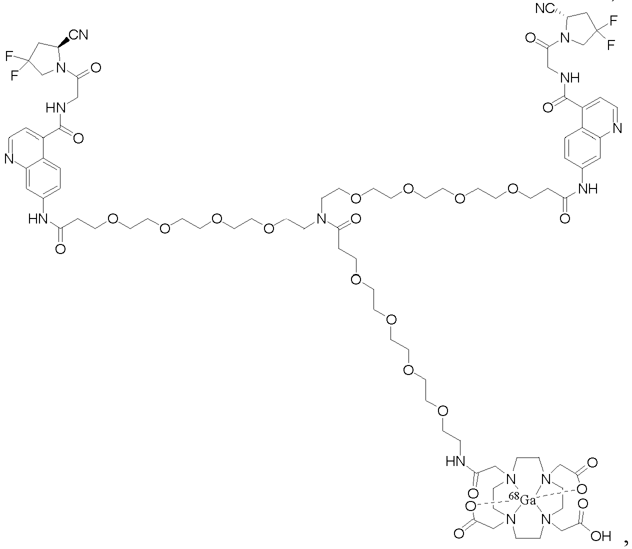 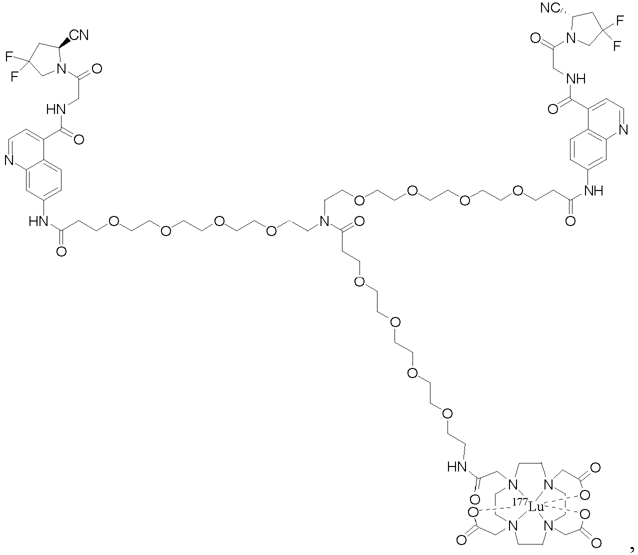 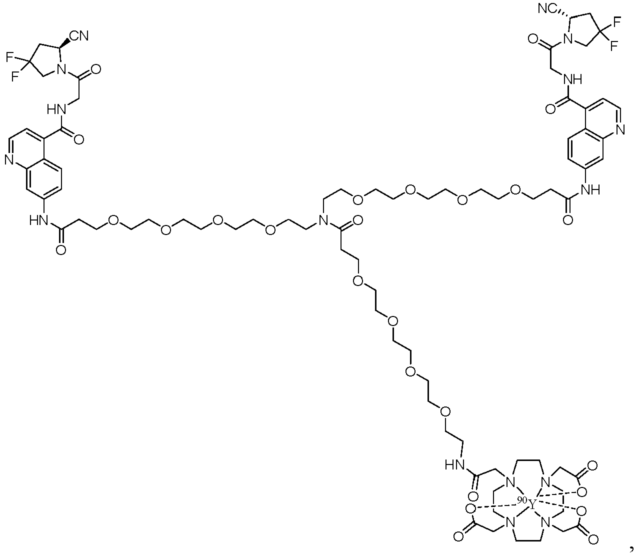 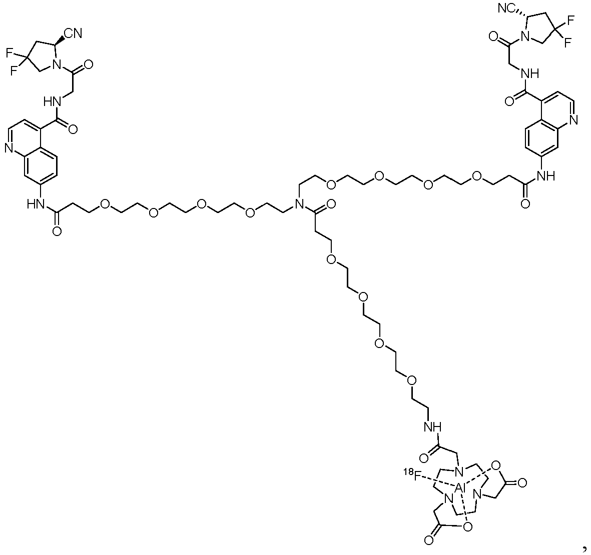 or a pharmaceutically acceptable salt, stereoisomer thereof.

10. A kit comprising the complex compound of any of claims 8-9 and a pharmaceutically acceptable carrier.

11. A method for the diagnosis of a disease **characterized by** overexpression of fibroblast activation proteinα (FAP-α) in a subject in need thereof, comprising the use of the complex compound of any of claims 8-9 or the kit of claim 10.

12. The method of claim 11, wherein the disease is selected from the group consisting of cancer, chronic inflammation, atherosclerosis, fibrosis, tissue remodeling, and keloid disorder.

13. The method of claim 12, wherein the disease is selected from the group consisting of breast cancer, pancreatic cancer, small intestine cancer, colon cancer, rectal cancer, lung cancer, head and neck cancer, ovarian cancer, liver cancer, esophageal cancer, gastric cancer, hypopharynx cancer, nasopharynx cancer, larynx cancer, myeloma, bladder cancer, cholangiocarcinoma, renal carcinoma, neuroendocrine tumor, oncogenic osteomalacia, sarcoma, carcinoma of unknown primary, thymus carcinoma, glioma, astrocytoma, cervix carcinoma, prostate cancer, and testicular cancer.

## Patentansprüche

1. Liganden-Verbindung der Formel (IC) 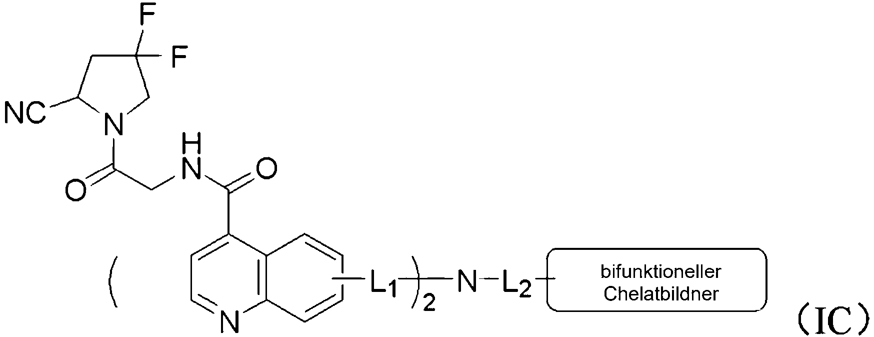 worin
L₁ ein funktionalisierter Linker -X₁-CO-C₁₋₆-Alkylen(-X₂-C₁₋₆-alkylen)ₘ- ist;
L₂ ein funktionalisierter Linker -CO-C₁₋₆-Alkylen-(X₃-C₁₋₆-alkylen-)ₙ-X₄- ist;
X₁, X₂, X₃, X₄ jeweils unabhängig aus der aus O, S, NH und NCH₃ bestehenden Gruppe ausgewählt sind, wobei das C₁₋₆-Alkylen gegebenenfalls mit Halogen, OH, NH₂, Oxo und/ oder Cyano substituiert ist;
m, n jeweils eine ganze Zahl, ausgewählt aus 0, 1, 2, 3, 4, 5, 6, 7, 8, 9 und 10 sind;
der bifunktionelle Chelatbildner aus der aus 6-(2-(Sulfobenzyliden)hydrazinyl)-nicotinsäure (HYNIC), Mercaptoacetyldiglycin (MAG₂), Mercaptoacetyltriglycin (MAG₃), 1,4,7,10-Tetraazacyclododecan-1,4,7,10-tetraessigsäure (DOTA), 1,4,7-Triazacyclononan-1,4,7-triessigsäure (NOTA), 1,4,8,11-Tetraazacyclotetradecan-1,4,8,11-tetraessigsäure (TETA), Ethylendiamintetraessigsäure (EDTA), Diethylentriamin-N,N,N',N',N"-pentaessig-säure (DTPA), 3,6,9,15-Tetraazabicyclo[9.3.1]pentadecan-1(15),11,13-trien-3,6,9-triessig-säure (PCTA), RESCA, 1,4,7-Triazacyclononan-1-glutarsäure-4,7-diessigsäure (NOTA-GA), 1,4,7,10-Tetraazacyclododecan-1-glutarsäure-4,7,10-triessigsäure (DOTA-GA), 1,4,8,11-Tetraazabicyclo[6,6,2]hexadecan-4,11-diessigsäure (CB-TE2A), 1,8-Diamino-3,6,10,13,16, 19-hexazabicyclo[6,6,6]eicosan (DiAmSar), 1-(4-Isothiocyanatophenyl)-3-[6,17-dihydroxy-7,10,18,21-tetraoxo-27-(N-acetylhydroxylamino)-6,11,17,22-tetraazaheptacosan] (DFO) bestehenden Gruppe ausgewählt ist;
oder ein pharmazeutisch annehmbares Salz oder Stereoisomer davon.

2. Liganden-Verbindung nach Anspruch 1, welche die Verbindung der Formel (IC-I), (IC-II), (IC-III) oder (IC-IV): 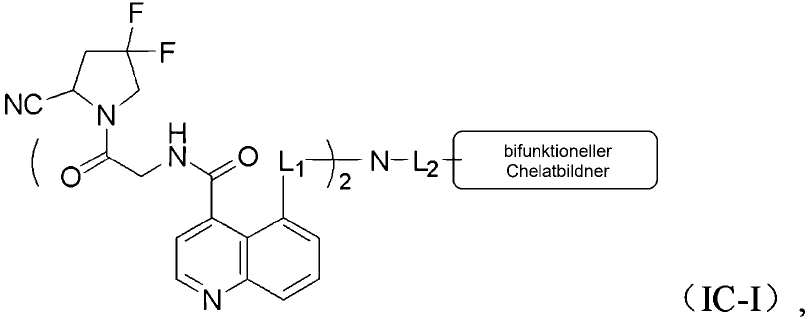 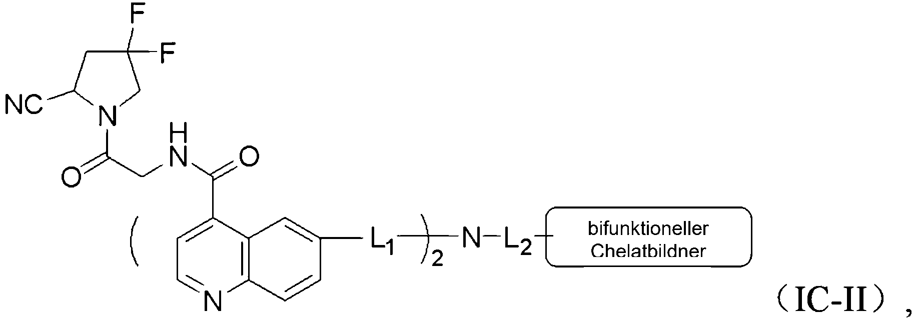 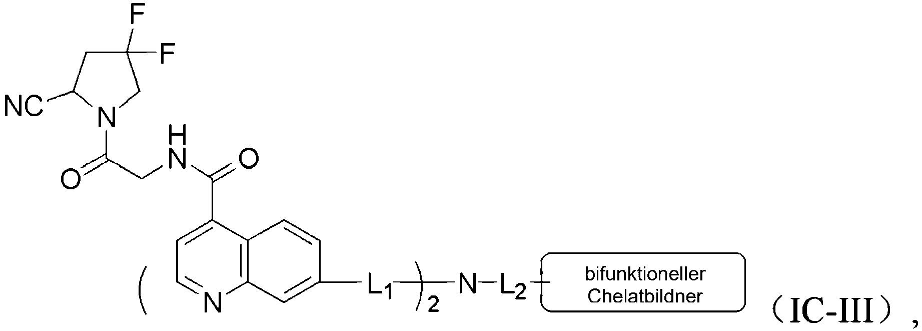 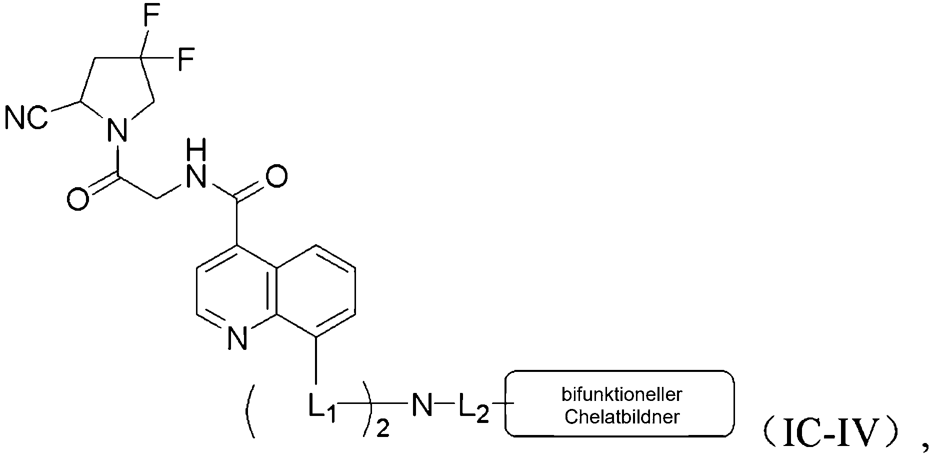
oder ein pharmazeutisch annehmbares Salz oder Stereoisomer davon ist,
worin L₁, L₂ und der bifunktionelle Chelatbildner wie in Anspruch 1 definiert sind.

3. Liganden-Verbindung nach Anspruch 1, welche die Verbindung der Formel (ID): 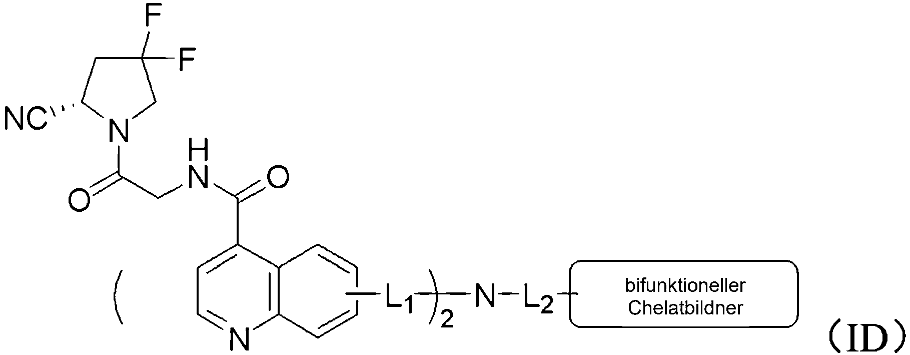
oder ein pharmazeutisch annehmbares Salz oder Stereoisomer davon ist,
worin L₁, L₂ und der bifunktionelle Chelatbildner wie in Anspruch 1 definiert sind.

4. Liganden-Verbindung nach Anspruch 3, welche die Verbindung der Formel (ID-1), (ID-II), oder (ID-IV): 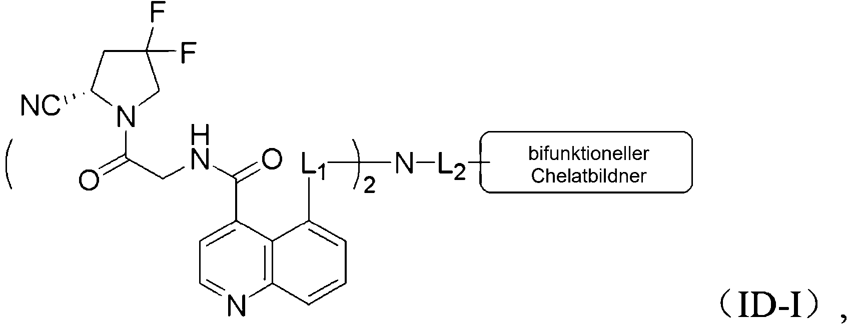 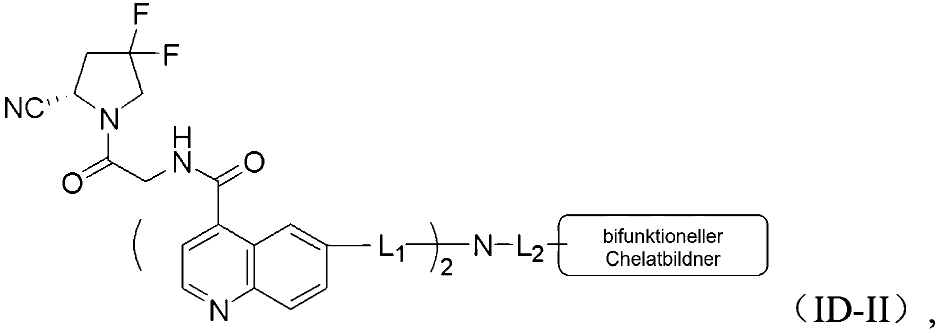 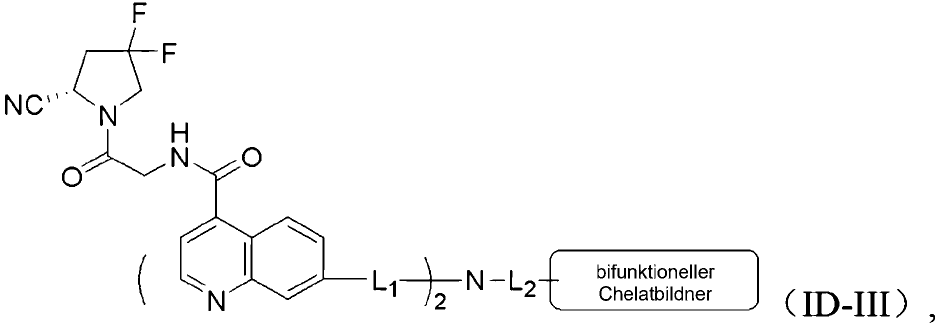 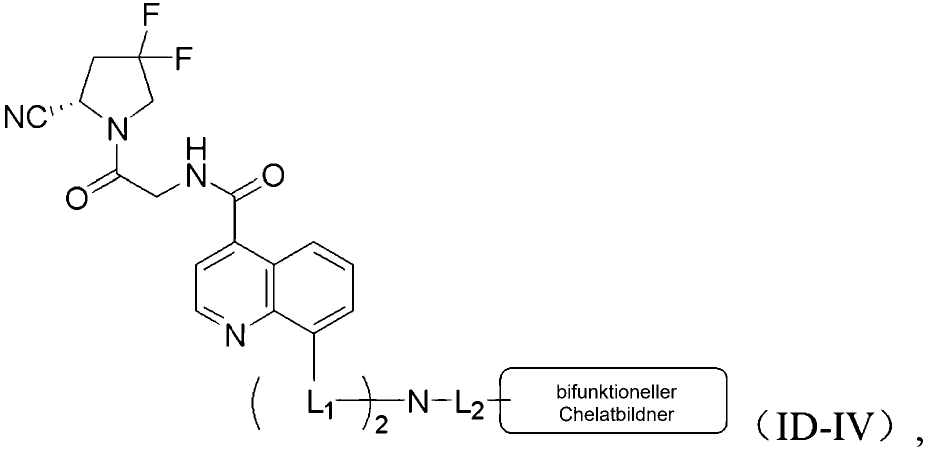
oder ein pharmazeutisch annehmbares Salz oder Stereoisomer davon ist,
worin L₁, L₂ und der bifunktionelle Chelatbildner wie in Anspruch 1 definiert sind.

5. Liganden-Verbindung nach einem der Ansprüche 1 bis 4, worin L₁ der funktionalisierte Linker -NHCO-C₁₋₆-Alkylen(-O-C₁₋₆-alkylen)ₘ-, -NHCO-C₁₋₆-AlkylenNH-C₁₋₆-alkylen)ₘ-, -NHCO-C₁₋₆-Alkylen(-NCH₃-C₁₋₆-Alkylen)ₘ-, -NCH₃-CO-C₁₋₆-Alkylen(-O-C₁₋₆-alkylen)ₘ-, NCH₃-CO-C₁₋₆-Alkylen(-NH-C₁₋₆-Alkylen)ₘ-, -NCH₃-CO-C₁₋₆-Alkylen(-NCH₃-C₁₋₆-alkylen)ₘ-, -OCO-C₁₋₆-Alkylen(-O-C₁₋₆-alkylen)ₘ-, -OCO-C₁₋₆-Alkylen(-NH-C₁₋₆-alkylen)ₘ- und/oder -OCO-C₁₋₆-Alkylen(-NCH₃-C₁₋₆-alkylen)ₘ- ist, wobei das Alkylen gegebenenfalls mit Halogen, OH, NH₂, Oxo und/oder Cyano substituiert ist und m eine ganze Zahl ist, die aus 1, 2, 3, 4, 5 und 6 ausgewählt ist;
L₂ der funktionalisierte Linker -CO-C₁₋₆-Alkylen-(NH-C₁₋₆-alkylen-)ₙ-NH-, -CO-C₁₋₆-Alkylen-(NH-C₁₋₆-alkylen-)ₙ-NCH₃-, -CO-C₁₋₆-Alkylen-(NH-C₁₋₆-alkylen-)ₙ-O-, -CO-C₁₋₆-Alkylen-(O-C₁₋₆-alkylen-)ₙ-NH-, -CO-C₁₋₆-Alkylen-(O-C₁₋₆-alkylen-)ₙ-NCH₃-, -CO-C₁₋₆-Alkylen-(O-C₁₋₆-alkylen-)ₙ-O-, -CO-C₁₋₆-Alkylen-(NCH₃-C₁₋₆-alkylen-)ₙ-NH-, -CO-C₁₋₆-Alkylen-(NCH₃-C₁₋₆-alkylen-)ₙ-NCH₃- und/oder -CO-C₁₋₆-Alkylen-(NCH₃-C₁₋₆-alkylen-)ₙ-O- ist, wobei das Alkylen gegebenenfalls mit Halogen, OH, NH₂, Oxo und/oder Cyano substituiert ist und n die ganze Zahl 1, 2, 3, 4, 5 oder 6 ist.

6. Liganden-Verbindung nach Anspruch 5, worin L₁ der funktionalisierte Linker -NHCO-CH₂CH₂-(-O-CH₂CH₂)₄- ist und L₂ der funktionalisierte Linker -CO-CH₂CH₂-(O-CH₂CH₂-)₄-NH-ist.

7. Liganden-Verbindung nach Anspruch 1, die aus den folgenden ausgewählt ist: 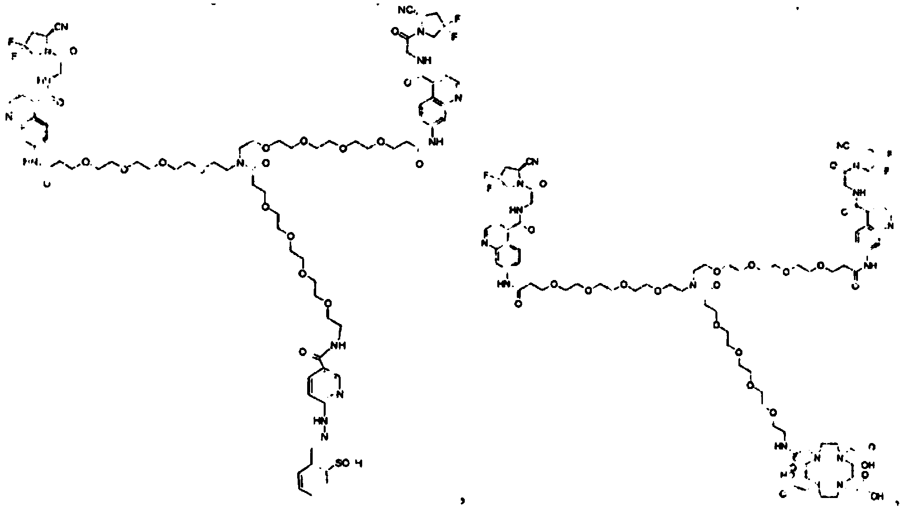 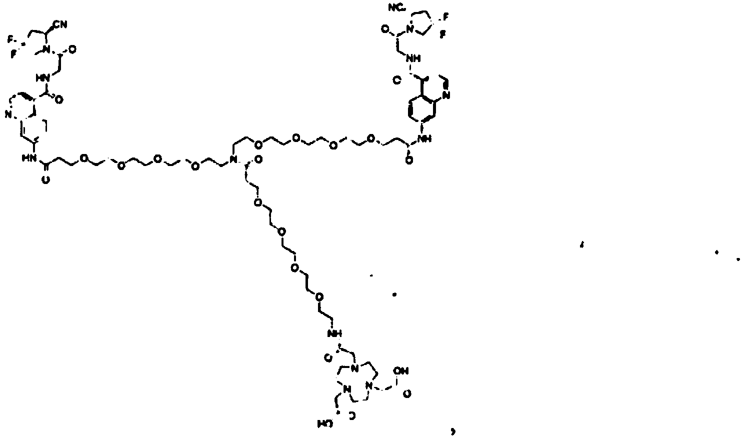 oder ein pharmazeutisch annehmbares Salz oder Stereoisomer davon ist.

8. Komplexverbindung, die eine Verbindung nach einem der Ansprüche 1 bis 7 und ein Radionuklid umfasst, wobei das Radionuklid über eine koordinative Bindung an den bifunktionellen Chelatbildner einer Verbindung nach einem der Ansprüche 1 bis 9 konjugiert ist, wobei das Radionuklid aus der aus α-Strahlung aussendenden Isotopen, β-Strahlung aussendenden Isotopen, γ-Strahlung aussendenden Isotopen, Auger-Elektronen aussendenden Isotopen und Röntgenstrahlen aussendenden Isotopen ausgewählt ist, wobei das Radionuklid vorzugsweise aus der aus ¹⁸F, ⁵¹Cr, ⁶⁷Ga, ⁶⁸Ga, ¹¹¹In, ⁹⁹mTc, ¹⁸⁶Re, ¹⁸⁸Re, ¹³⁹La, ¹⁴⁰La, ¹⁷⁵Yb, ¹⁵³Sm, ¹⁶⁶Ho, ⁸⁸Y, ⁹⁰Y, ¹⁴⁹Pm, ¹⁷⁷Lu, ⁴⁷Sc, ²¹²Bi, ²¹³Bi, ⁷²As, ¹²³I, ¹²⁴I, ¹³¹I, ²¹¹At, ²⁰¹Tl, ²¹²Pb, ⁶⁴Cu, ⁶⁷Cu, ¹⁹⁸Au, ²²⁵Ac, ²²³Ra und ⁸⁹Sr bestehenden Gruppe ausgewählt ist.

9. Komplexverbindung nach Anspruch 8, die aus den folgenden ausgewählt ist: 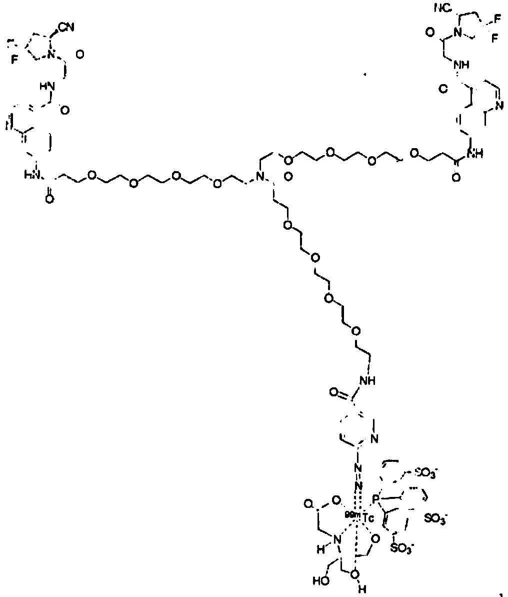 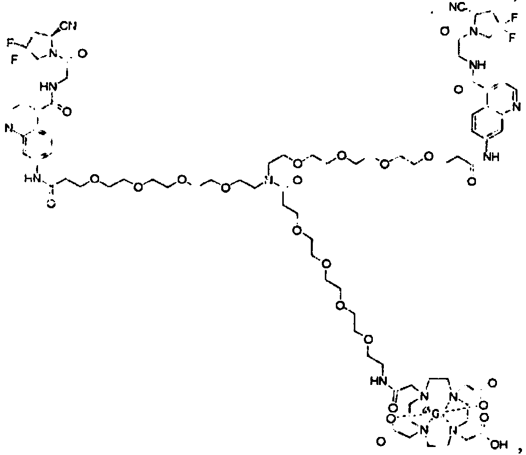 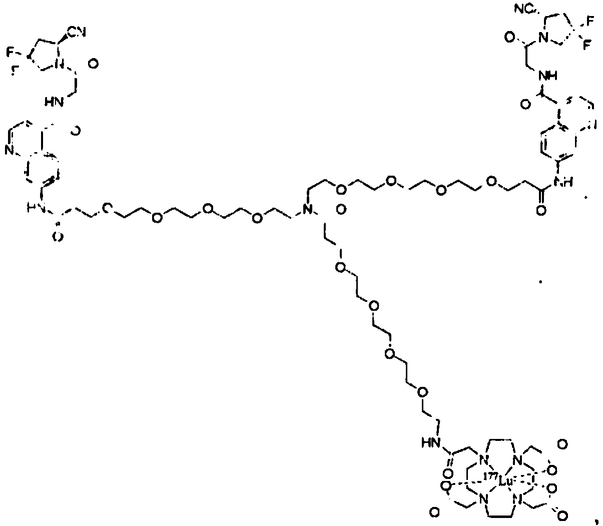 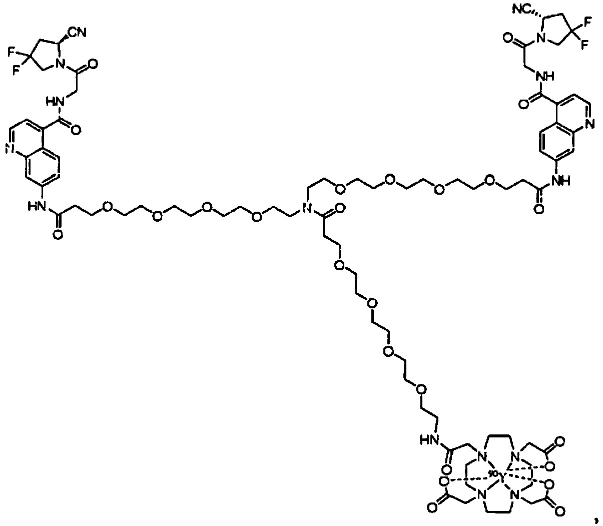 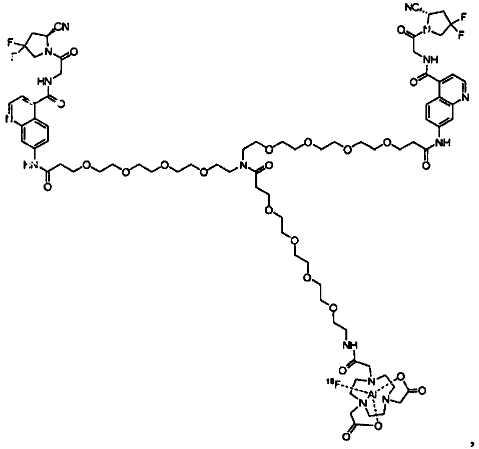 oder ein pharmazeutisch annehmbares Salz oder Stereoisomer davon ist.

10. Set, das eine Komplexverbindung nach einem der Ansprüche 8 bis 9 und einen pharmazeutisch annehmbaren Träger umfasst.

11. Verfahren zur Diagnose einer Erkrankung, die durch die Überexpression des Fibroblasten-Aktivierungs-Proteins α (FAP-α) gekennzeichnet ist, in einem bedürftigen Individuum, das die Verwendung einer Komplexverbindung nach einem der Ansprüche 8 oder 9 oder eines Sets nach Anspruch 10 umfasst.

12. Verfahren nach Anspruch 11, wobei die Erkrankung aus der aus Krebs, chronischer Entzündung, Atherosklerose, Fibrose, Geweberemodellierung und Keloidstörung bestehenden Gruppe ausgewählt ist.

13. Verfahren nach Anspruch 12, wobei die Erkrankung aus der aus Brustkrebs, Pankreaskrebs, Dünndarmkrebs, Dickdarmkrebs, Rektumkrebs, Lungenkrebs, Kopf- und Halskrebs, Eierstockkrebs, Leberkrebs, Speiseröhrenkrebs, Magenkrebs, Hypopharynxkrebs, Nasopharynxkrebs, Larynxkrebs, Myelom, Blasenkrebs, Cholangiokarzinom, Nierenkarzinom, neuroendokriner Tumor, onkogenen Osteomalazie, Sarkom, Karzinom unbekannter Ursache, Thymuskarzinom, Gliom, Astrozytom, Zervixkarzinom, Prostatakrebs und Hodenkrebs bestehenden Gruppe ausgewählt ist.

## Revendications

1. Composé ligand de formule (IC) 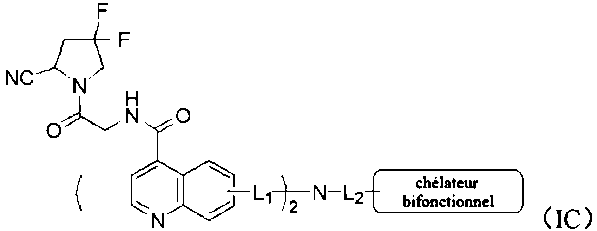 dans lequel,
L₁ est un lieur fonctionnalisé de -X₁-CO-alkylène en C_{1- 6}(-X₂-alkylène en C₁₋₆)ₘ- ;
L₂ est un lieur fonctionnalisé de -CO-alkylène en C₁₋₆-(X₃-alkylène en C₁₋₆)ₘ-X₄- :
X₁, X₂, X₃ et X₄ sont choisis indépendamment dans le groupe constitué par O, S, NH et NCH₃ ; le groupe alkylène en C₁₋₆ est éventuellement substitué par un groupe halogéno, OH, NH₂, un groupe oxo et/ou un groupe cyano ;
m, n sont chacun un nombre entier choisi parmi 0, 1, 2, 3, 4, 5, 6, 7, 8, 9 ou 10 ;
le chélateur bifonctionnel est choisi dans le groupe constitué par l'acide 6-(2-(sulfobenzylidène)hydrazinyl)nicotinique (HYNIC), la mercaptoacétyldiglycine (MAG₂), la mercaptoacétyltriglycine (MAG₃), l'acide 1,4,7,10-tétraazacyclododécane-1,4,7,10-tétraacétique (DOTA), l'acide 1,4,7-triazacyclononane-1,4,7-triacétique (NOTA), l'acide 1,4,8,11-tétraazacyclotétradécane-1,4,8,11-tétraacétique (TETA), l'acide éthylènediaminetétraacétique (EDTA), l'acide diéthylènetriamine-N,N,N',N',N"-pentaacétique (DTPA), le 3,6,9,15-tétraazabicyclo[9.3.1]pentadécane-1(15),11,13-triène-3,6,9-triacétique (PCTA), RESCA, l'acide 1,4,7-triazacyclononane-1-glutarique-4,7-diacétique (NOTA-GA), l'acide 1,4,7,10-tétraazacyclododécane-1-glutarique-acide 4,7,10-triacétique (DOTA-GA), l'acide 1,4,8,11-tétraazabicyclo[6,6,2]hexadécane-4,11-diacétique (CB-TE2A), le 1,8-diamino-3,6,10,13,16,19-hexazabicyclo[6,6,6]éicosane (DiAmSar), le 1-(4-isothiocyanatophényl)-3-[6,17-dihydroxy-7,10,18,21-tétraoxo-27-(N-acétylhydroxylamino)-6,11,17,22-tétraazaheptacosane] (DFO) ;
ou un sel pharmaceutiquement acceptable, un stéréoisomère de celui-ci.

2. Composé ligand selon la revendication 1, qui est le composé de formule (IC-I), (IC-II), (IC-III) ou (IC-IV), 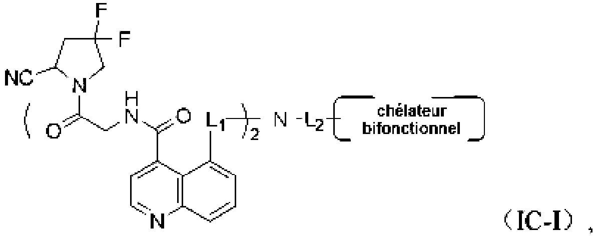 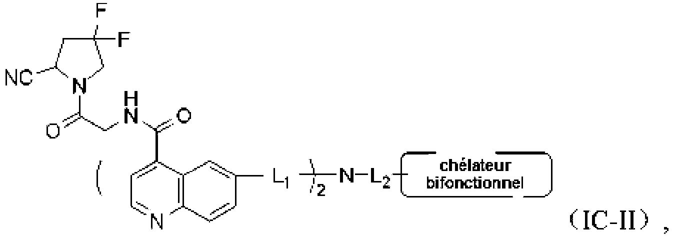 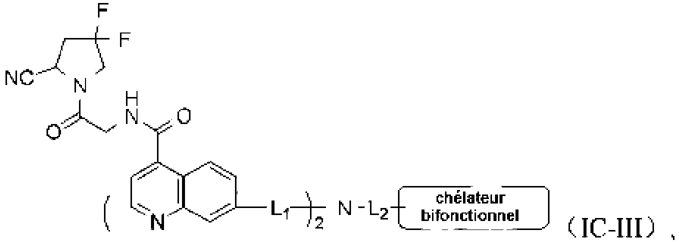 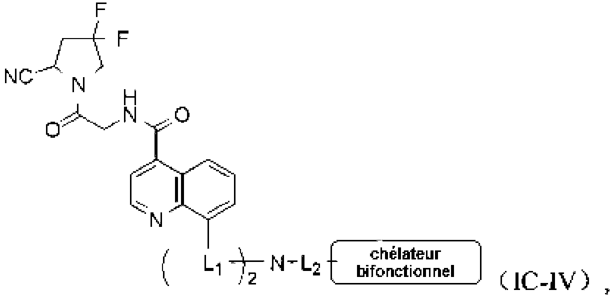
ou un sel pharmaceutiquement acceptable, un stéréoisomère de celui-ci ;
dans lequel L₁, L₂ et le chélateur bifonctionnel sont tels que définis dans la revendication 1.

3. Composé ligand selon la revendication 1, qui est le composé de formule (ID), 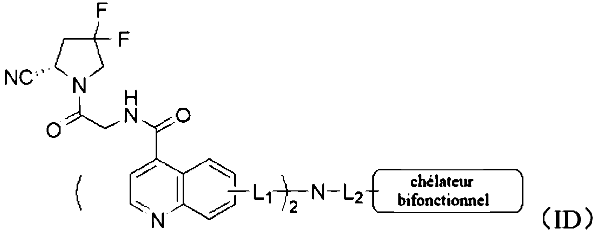
ou un sel pharmaceutiquement acceptable, un stéréoisomère de celui-ci ;
dans lequel L₁, L₂ et le chélateur bifonctionnel sont tels que définis dans la revendication 1.

4. Composé ligand selon la revendication 3, qui est le composé de formule (ID-I), (ID-II), (ID-III) ou (ID-IV), 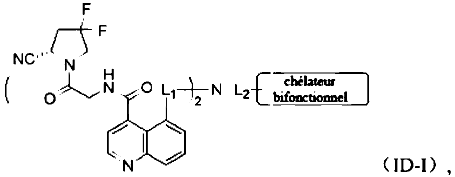 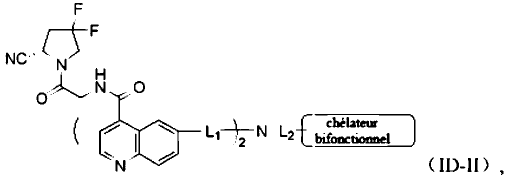 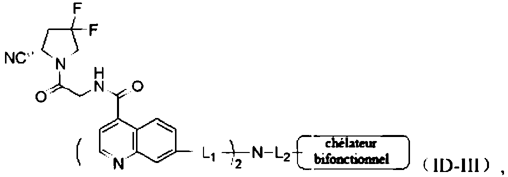 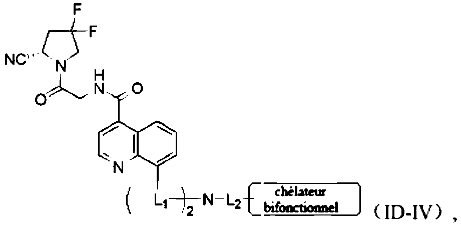
ou un sel pharmaceutiquement acceptable, un stéréoisomère de celui-ci ;
dans lequel L₁, L₂, le chélateur bifonctionnel sont tels que définis dans la revendication 1.

5. Composé ligand selon l'une quelconque des revendications 1 à 4, dans lequel L₁ est le lieur fonctionnalisé de -NHCO-alkylène en C₁₋₆(-O-alkylène en C₁₋₆) ₘ-, -NHCO-alkylène en C₁₋₆ (-NH-alkylène en C₁₋₆) ₘ-, -NHCO-alkylène en C₁₋₆(-NCH₃-alkylène en C₁₋₆)ₘ-, -NCH₃-CO-alkylène en C₁₋₆ (-O-alkylène en C₁₋₆) ₘ-, -NCH₃-CO-alkylène en C₁₋₆(-NH-alkylène en C₁₋₆)ₘ-, -NCH₃-CO-alkylène en C₁₋₆ (-NCH₃-alkylène en C₁₋₆)m-, -OCO-alkylène en C₁₋₆(-O-alkylène en C₁₋₆)ₘ-, -OCO-alkylène en C₁₋₆(-NH-alkylène en C₁₋₆)ₘ- et/ou -OCO-alkylène en C₁₋₆(-NCH₃-alkylène en C₁₋₆) ₘ-, dans lequel le groupe alkylène est éventuellement substitué par un groupe halogéno, OH, NH₂, un groupe oxo et/ou un groupe cyano, m est un nombre entier choisi parmi 1, 2, 3, 4, 5 ou 6 ;
L₂ est le lieur fonctionnalisé de -CO-alkylène en C₁₋₆-(NH-alkylène en C₁₋₆)ₙ-NH-, -CO-alkylène en C₁₋₆- (NH-alkylène en C₁₋₆)ₙ-NCH₃-, -CO-alkylène en C₁₋₆-(NH-alkylène en C₁₋₆)ₙ-O-, -CO-alkylène en C₁₋₆-(O-alkylène en C₁₋₆)ₙ-NH-, -CO-alkylène en C₁₋₆-(O-alkylène en C₁₋₆)ₙ-NCH₃-, -CO-alkylène en C₁₋₆-(O-alkylène en C₁₋₆)ₙ-O-, -CO-alkylène en C₁₋₆- (NCH₃-alkylène en C₁₋₆)ₙ-NH-, -CO-alkylène en C₁₋₆-(NCH₃-alkylène en C₁₋₆)ₙ-NCH₃-et/ou -CO-alkylène en C₁₋₆-(NCH₃-alkylène en C₁₋₆)ₙ-O-, dans lequel le groupe alkylène est éventuellement substitué par un groupe halogéno, OH, NH₂, un groupe oxo et/ou un groupe cyano, n est un nombre entier choisi parmi 1, 2, 3, 4, 5 ou 6.

6. Composé ligand selon la revendication 5, dans lequel L₁ est le lieur fonctionnalisé de -NHCO-CH₂CH₂- (-O-CH_{2C}H₂)₄- ; L₂ est le lieur fonctionnalisé de -CO-CH₂CH₂- (O-CH₂CH₂)₄-NH-.

7. Composé ligand selon la revendication 1, choisi parmi : 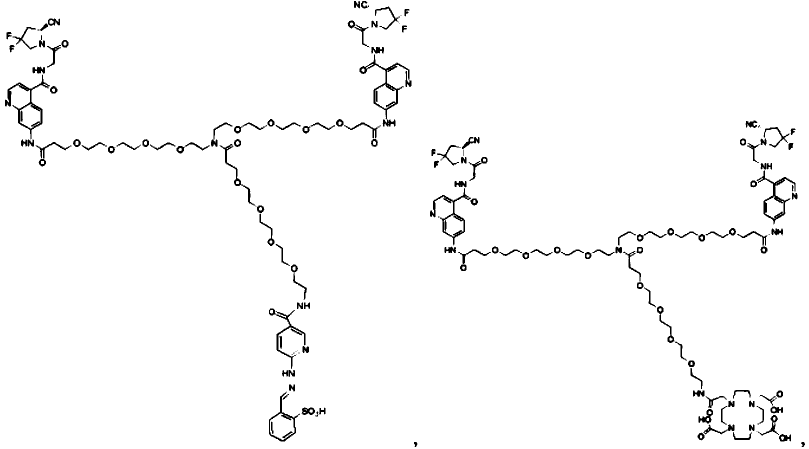 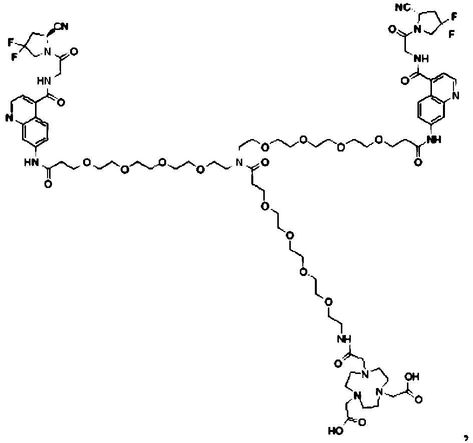 ou un sel pharmaceutiquement acceptable, un stéréoisomère de celui-ci.

8. Composé complexe comprenant le composé selon l'une quelconque des revendications 1 à 7 et un radionucléide, dans lequel le radionucléide est conjugué au chélateur bifonctionnel du composé selon l'une quelconque des revendications 1 à 9 par une liaison de coordination, dans lequel le radionucléide est choisi dans le groupe constitué par les isotopes émetteurs de rayonnement alpha, les isotopes émetteurs de rayonnement bêta, les isotopes émetteurs de rayonnement gamma, les isotopes émetteurs d'électrons Auger, les isotopes émetteurs de rayons X ; de préférence, le radionucléide est choisi dans le groupe constitué par ¹⁸F, ⁵¹Cr, ⁶⁷Ga, ⁶⁸Ga, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶HO, ⁸⁸Y, ⁹⁰Y, ¹⁴⁹Pm, ¹⁷⁷Lu, ⁴⁷Sc, ²¹²Bi, ²¹³Bi, ⁷²As, ¹²³I, ¹²⁴I, ¹³¹I, ²¹¹At, ²⁰¹Tl, ²¹²Pb, ⁶⁴Cu, ⁶⁷Cu, ¹⁹⁸Au, ²²⁵Ac, ²²³Ra ou ⁸⁹Sr.

9. Composé complexe selon la revendication 8, qui est choisi parmi : 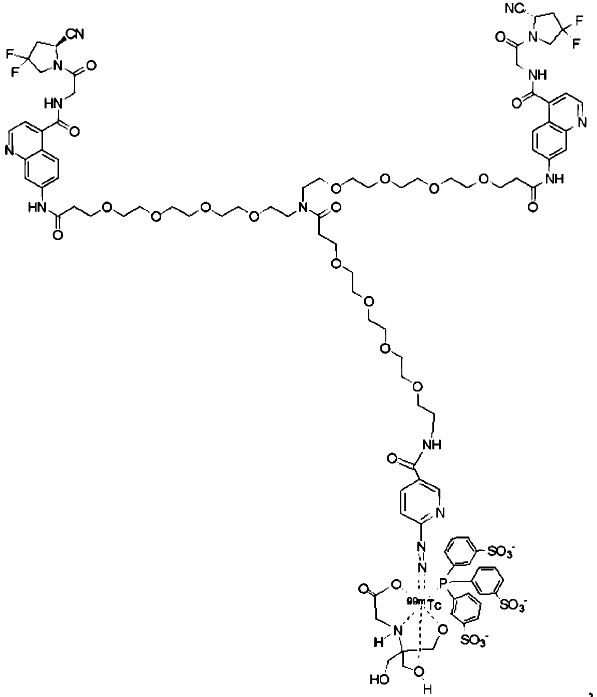 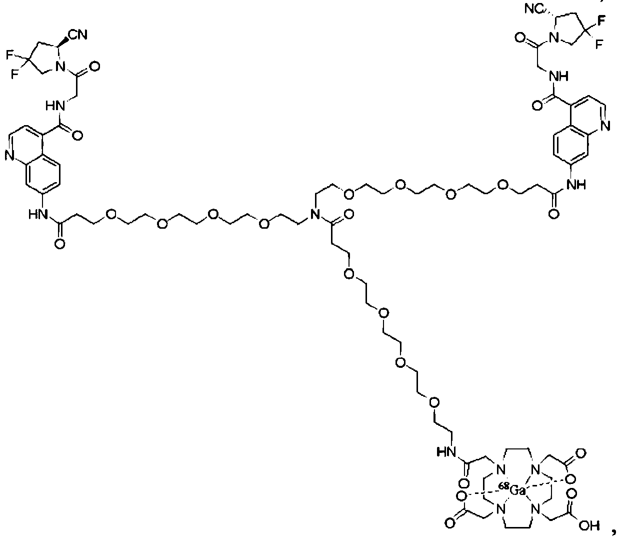 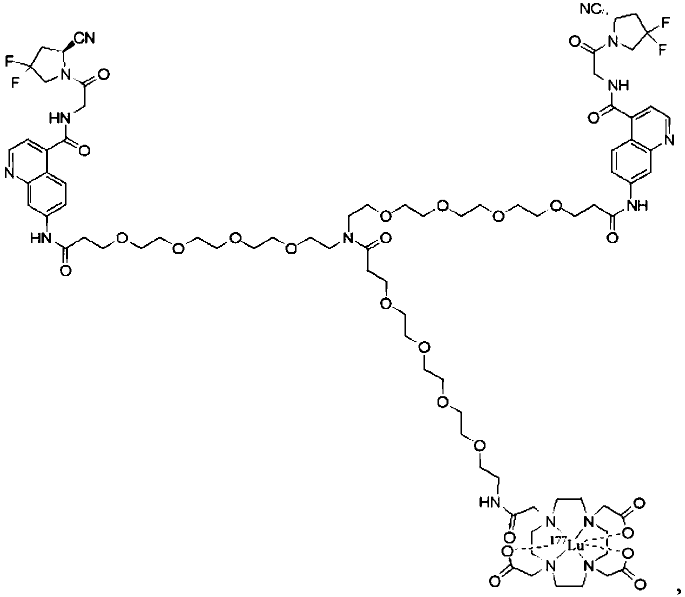 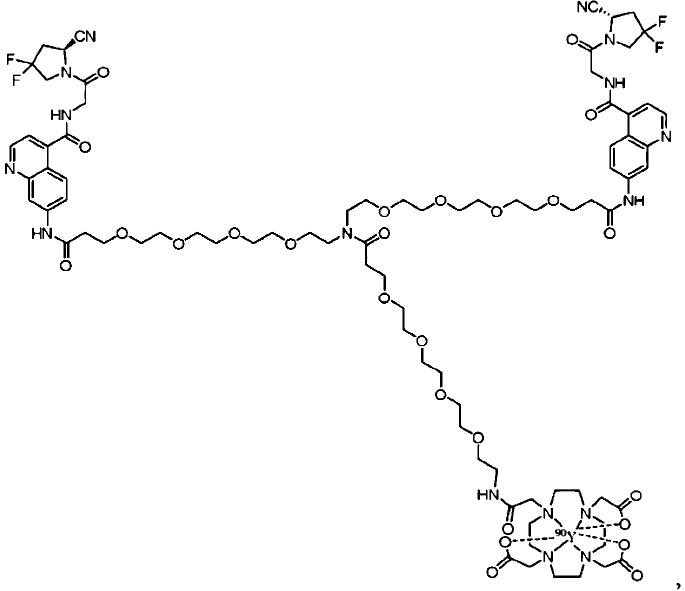 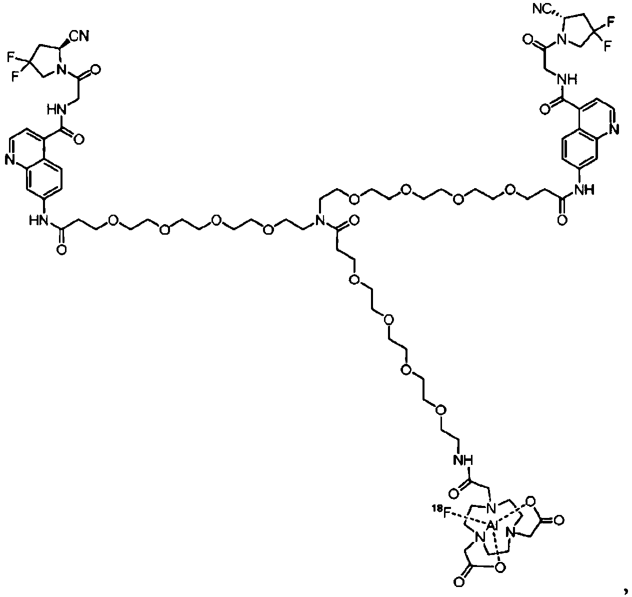 ou un sel pharmaceutiquement acceptable, un stéréoisomère de celui-ci.

10. Kit comprenant le composé complexe selon l'une quelconque des revendications 8 à 9 et un véhicule pharmaceutiquement acceptable.

11. Méthode de diagnostic d'une maladie **caractérisée par** une surexpression de la protéine d'activation des fibroblastes α (FAP-α) chez un sujet qui en a besoin, comprenant l'utilisation du composé complexe selon l'une quelconque des revendications 8 à 9 ou du kit selon la revendication 10.

12. Méthode selon la revendication 11, dans laquelle la maladie est choisie dans le groupe constitué par le cancer, l'inflammation chronique, l'athérosclérose, la fibrose, le remodelage tissulaire et le trouble chéloïdien.

13. Méthode selon la revendication 12, dans laquelle la maladie est choisie dans le groupe constitué par le cancer du sein, le cancer du pancréas, le cancer de l'intestin grêle, le cancer du côlon, le cancer du rectum, le cancer du poumon, le cancer de la tête et du cou, le cancer de l'ovaire, le cancer du foie, le cancer de l'œsophage, le cancer de l'estomac, le cancer de l'hypopharynx, le cancer du nasopharynx, le cancer du larynx, le myélome, le cancer de la vessie, le cholangiocarcinome, le carcinome rénal, la tumeur neuroendocrinienne, l'ostéomalacie oncogénique, le sarcome, le carcinome d'origine primitive inconnue, le carcinome du thymus, le gliome, l'astrocytome, le carcinome du col de l'utérus, le cancer de la prostate et le cancer du testicule.
